# EUROPEAN PATENT SPECIFICATION

(11) **EP 2 751 071 B1**
(45) Date of publication and mention of the grant of the patent: **27.11.2019**
(21) Application number: 12828585.5
(22) Date of filing: 31.08.2012
(51) Int. Cl.: C07C 233/20, A61K 31/16, A61K 31/19, C07H 13/04, A61K 31/70, A61K 31/60, A61K 31/397, A61P 29/00, A61P 3/00, A61P 9/10, A61P 3/06, A61P 3/10, A61P 1/16, A61P 37/06, A61P 25/28, A61P 19/02, A61P 17/06, A61P 1/00, A61P 25/16, C07C 323/41, A61K 45/06

(54) **FATTY ACID AMIDES, COMPOSITIONS AND METHODS OF USE**
FETTSÄUREAMIDE, ZUSAMMENSETZUNGEN DAMIT UND ANWENDUNGSVERFAHREN DAFÜR
AMIDES D'ACIDE GRAS, COMPOSITIONS ET PROCÉDÉS D'UTILISATION

(30) Priority: 31.08.2011 US 201161529760 P; 09.02.2012 US 201261596916 P
(43) Date of publication of application: 09.07.2014
(73) Proprietor: Milne, Jill C., Brookline, Massachusetts 02146 (US); Jirousek, Michael R., Cambridge, Massachusetts 02142 (US); Bemis, Jean E., Arlington, Massachusetts 02476 (US); Vu, Chi B., Arlington, Massachusetts 02474 (US); Ting, Amal, Newton, Massachusetts 02459 (US)
(72) Inventor: Milne, Jill C., Brookline, Massachusetts 02146 (US); Jirousek, Michael R., Cambridge, Massachusetts 02142 (US); Bemis, Jean E., Arlington, Massachusetts 02476 (US); Vu, Chi B., Arlington, Massachusetts 02474 (US); Ting, Amal, Newton, Massachusetts 02459 (US)
(74) Representative: Grund, Martin
(86) International application number: PCT/US2012/053452
(87) International publication number: WO 2013/033602

(56) References cited:
- US-A1- 2010 016 328
- US-A1- 2010 168 054
- US-A1- 2010 184 730
- US-A1- 2011 053 990
- US-A1- 2011 082 156
- US-A1- 2011 082 202
- US-A1- 2011 082 210
- US-A1- 2011 172 240
- US-A1- 2011 172 240
- HUNG THE DANG ET AL: "Evaluation of endogenous fatty acid amides and their synthetic analogues as potential anti-inflammatory leads", BIOORGANIC & MEDICINAL CHEMISTRY, PERGAMON, GB, vol. 19, no. 4, 22 December 2010 (2010-12-22), pages 1520-1527, XP028363744, ISSN: 0968-0896, DOI: 10.1016/J.BMC.2010.12.046 [retrieved on 2010-12-30]
- MCCAUGHAN B ET AL: "A potential new prodrug for the treatment of cystinosis: Design, synthesis and in-vitro evaluation", BIOORGANIC & MEDICINAL CHEMISTRY LETTERS, PERGAMON, AMSTERDAM, NL, vol. 18, no. 5, 1 March 2008 (2008-03-01), pages 1716-1719, XP022683125, ISSN: 0960-894X, DOI: 10.1016/J.BMCL.2008.01.039 [retrieved on 2008-01-18]

## Description

Oily cold water fish, such as salmon, trout, herring, and tuna are the source of dietary marine omega-3 fatty acids, with eicosapentaenoic acid (EPA) and docosahexaenoic acid (DHA) being the key marine derived omega-3 fatty acids. Omega-3 fatty acids have previously been shown to improve insulin sensitivity and glucose tolerance in normoglycemic men and in obese individuals. Omega-3 fatty acids have also been shown to improve insulin resistance in obese and non-obese patients with an inflammatory phenotype. Lipid, glucose, and insulin metabolism have been shown to improve in overweight hypertensive subjects through treatment with omega-3 fatty acids. Omega-3 fatty acids (EPA/DHA) have also been shown to decrease triglycerides and to reduce the risk for sudden death caused by cardiac arrhythmias in addition to improve mortality in patients at risk of a cardiovascular event. Omega-3 fatty acids have also been taken as dietary supplements part of therapy used to treat dyslipidemia, and anti-inflammatory properties. A higher intake of omega-3 fatty acids lower levels of circulating TNF-α and IL-6, two of the cytokines that are markedly increased during inflammation processes (Chapkin et al, Prostaglandins, Leukot Essent Fatty Acids 2009, 81, p. 187-191; Duda et al, Cardiovasc Res 2009, 84, p. 33-41). In addition, a higher intake of omega-3 fatty acids has been shown to increase levels of the well-characterized anti-inflammatory cytokine IL-10 (Bradley et al, Obesity (Silver Spring) 2008, 16, p. 938-944). A recent study (Wang et al, Molecular Pharmaceutics 2010,7, p. 2185-2193) has demonstrated that DHA could also induce the Nrf2 and the Nrf2-target gene Heme-oxygenase 1 (HO-1) and this pathway could play a significant role in suppressing LPS-mediated inflammation. A number of studies have now indicated that DHA could play a significant role in cancer (For reviews see: Gleissman, H. et al Experimental Cell Research 2010, 316, p. 1365-73; Bougnoux, P. et al Progress in Lipid Research 2010, 49, p. 76-86; Spencer, L. et al, Eur. J. Cancer 2009, 45, p. 2077-86; Serini, S. et al Apoptosis 2009, 14, p. 135-152; Browever, I. A. Prostaglandins, Leukotrienes and Essential Fatty Acids 2008, 79, p. 97-99). For instance, DHA was able to induce p53-dependent growth inhibition of transformed colon and lung carcinomas (Kikawa et al, J. of Cancer Science and Therapy, 2011, 3, p. 1-4). DHA has also been shown to prevent breast cancer cell metastasis to bone in a mouse model utilizing MDA-MB-231 human breast cancer cells (Mandal et al, Biochem. & Biophys. Res. Communications 2010, 402, p. 602-607).

Chronic oxidative stress and inflammation have now been linked to the development and progression of a number of debilitating diseases. Some of these diseases include renal failure, heart failure, atherosclerosis, osteoporosis, cancer, chronic obstructive pulmonary disease (COPD), Parkinson's disease and Alzheimer's disease. Activation of the Nrf2 pathway in order to resolve this chronic oxidative stress and inflammation appears to be a particularly promising new therapeutic approach (For a review see Gozzelino, R. et al Annu. Rev. Pharmacol. Toxicol. 2010, 50, p. 323-54). For instance, small molecule activators of Nrf2 have now been shown to be effective in the cisplatin-induced nephrotoxicity mouse model (Aleksunes et al, J. Pharmacology & Experimental Therapeutics 2010, 335, p. 2-12), the transgenic Tg19959 mouse model of Alzheimer's disease (Dumont et al, J. Neurochem. 2009, 109, p. 502-12), the mouse model for COPD (Sussan, T. E. et al Proc. Natl. Acad. Sci. USA 2009, 106, p. 250-5), and the murine 4T1 breast tumor model (Ling, X. et al Cancer Res. 2007, 67, p. 4210-8). Recent data suggested that DHA and EPA can potentially mitigate the progression of many diseases in which oxidative stress represents a common underlying cause (Gao et al, J. Biological Chem. 2007, 282, issue 4, p. 2529-37; Wang et al, Mol. Pharmaceutics 2010, 7, issue 6, p. 2185-2193). The oxidized form of omega-3 fatty acids can presumably react directly with Keap1, a negative regulator of Nrf2, in order to induce Nrf2 gene expression.

There are many amines that can be covalently coupled with omega-3 fatty acids to produce fatty acid amides with improved biological activity. The fatty acid amides disclosed herein have been designed to be stable in the plasma. However, in targeted tissues, intracellular enzymes will hydrolyze the fatty acid amides into the individual components, i.e. the omega-3 fatty acid and the amine moiety. In some embodiments, certain amine moieties do not necessarily display any significant biological activity. However, when these amines are coupled to an omega-3 fatty acid, the resulting fatty acid amides can be delivered into targeted tissues more efficiently to produce a biological effect that cannot be replicated by administering the fatty acid alone or in non covalent combination with the amine portion. In some embodiments, certain "PEGylated" amines are used to form fatty acid amides. "PEGylation" is a common technique of incorporating polyethylene glycol (PEG) chains into a molecule to improve its hydrophobicity and pharmacokinetic profile (Veronese et al Drug Discovery Today 2005, 10, p. 1451-8). When omega-3 fatty acids are coupled to a "PEGylated" amine, the resulting fatty acid amides can have an improved circulatory time and pharmacokinetic profile, in addition to being delivered into targeted tissues more efficiently than the corresponding acid. In some embodiments, the amine component itself displays significant biological activity. When these biologically active amines are coupled to an omega-3 fatty acid, the resulting fatty acid amides will produce a biological effect that cannot be replicated by administering the individual components or a noncovalent combination of the individual components. In some instances, the biological activity can be synergistic. In other instances, the side effects of the biologically active amines can be reduced since the individual components of the fatty acid amides are only released inside targeted tissues by hydrolysis involving various intracellular enzymes. Non-limiting examples of biologically active amine components are cystamine, cysteamine, glucosamine, dexpramipexole, sapropterin, N-acylated cysteine, pencillamine, triethylenetetramine, 4-aminopyridine, fingolimod, and pramipexole.

Cysteamine has been used extensively to treat nephropathic cystinosis, an orphan genetic lysosomal storage disorder characterized by a massive accumulation of crystalline cystine within cells. Treatment of nephropathic cystinotic fibroblasts with cysteamine has been shown to significantly reduce the excess cystine present in these cells (For a review, see Gahl et al N. Engl. J. Med. 2002, 347, p. 111-121). Cystamine, the disulfide form of cysteamine, has also been found to be useful in reducing cystine level in nephropathic cystinotic fibroblasts (Thoene et al Proc. Roy. Soc. Med. 1977, 70, Suppl. 3, p. 37-40). In addition, cysteamine and cystamine have been found to be neuroprotective in the YAC128 mouse model of Huntington's disease (Van Raamsdonk et al J. Neurochem. 2005, 95, p. 210-220). Fatty acid amide derivatives consisting of an omega-3 fatty acid that has been covalently liked to either cystamine or cysteamine can have an additional anti-inflammatory and neuroprotective effect that cannot be replicated by administering the individual components or the combination of the individual components.

Glucosamine has been used clinically to reduce the debilitating symptoms of osteoarthritis. In terms of mechanism of action, the anti-inflammatory properties of glucosamine can be partly attributed to the inhibition of prostaglandin E2 (PGE2) synthesis through the reduction of COX-2 (Kapor et al J. Rheumatology 2012, 39, p. 635-644). In addition, treatment with glucosamine can also up-regulate the gene expression of heme oxygenase (HO-1), an important protective cellular mechanism toward reactive oxygen species (Valvasan et al Rheumatology 2008, 47, p. 31-35). Fatty acid/glucosamine amide derivatives, created by covalently linking an omega-3 fatty acid to glucosamine via an amine linker group, can have synergistic activity along both the PGE2 pathway and the antioxidative Nrf2 pathway.

Dexpramipexole is a low molecular weight, water-soluble compound that has recently been shown to be clinically effective in treating amyotrophic lateral sclerosis (ALS) (Cudkowicz et al Nat. Med. 2011, 17, p. 1652-1657). In a phase 2 trial, ALS patients treated with Dexpramipexole showed a dose-related slowing of symptom progression as well as increased survival time. Preliminary studies into the mechanism of action appear to indicate that dexpramipexole can prevent mitochondrial ROS generation and thereby impart some neuroprotective effects (Ferrari-Toninelli et al BMC Pharmacology 2010, 10, p. 1-6). Because of this anti-oxidant and neuroprotective effect, a covalent amide derivative between an omega-3 fatty acid and dexpramipexole can have a synergistic effect that cannot be duplicated by administering the individual components or a combination of the components.

Phenylketonuria (PKU) is metabolic disorder that results from lack of enzymes that are needed to convert phenylalanine to tyrosine. If left untreated, the excessive buildup of phenylalanine can lead to mental retardation, speech impediments, seizures and behavioral abnormalities. Children with PKU often need supplementation with DHA for cognitive function and development. In patients with PKU, treatment with sapropterin has been shown to be effective in lowering blood level of phenylalanine. A fatty acid sapropterin amide can allow for more effective delivery of sapropterin to the liver where a high level of phenylalanine hydroxylase is expressed. In addition, with fatty acid sapropterin amides, DHA can also be delivered more effectively to cells to enhance cognitive function and development.

4-Aminopyridine and fingolimod are amine containing compounds that have been found to be useful in treating relapsing remitting multiple sclerosis. A covalent omega-3 fatty acid conjugate of 4-aminopyridine or fingolimod will have additional anti-inflammatory and neuroprotective effects that cannot be replicated by administering the individual components or the combination of the components. N-acylated cysteine, tiopronin and penicillamine are some thiol containing compounds that have been used clinically to treat Wilson's disease, cystinuria and to thin the thick mucus present in cystic fibrosis patients. Fatty acid amide derivatives containing these thiol compounds will allow better tissue targeting and reduce side effects.

US 2011/082210 A1 discloses fatty acid fibrate derivatives and their use in the treatment of metabolic diseases. US 2011/172240 A1 discloses fatty acid fumarate derivatives and their use in the treatment of cancer, metabolic disorders, and neurodegenerative disorders. McCaughan et al. (Bioorganic and Medical Chemistry Letters 2008, 18(5):1716-1719) discloses ((((carboxamido)ethyl)disulfanyl)ethyl)carboxamides for use in the treatment of nephropathic cystinosis.

### SUMMARY OF THE INVENTION

The invention is defined by the appended claims.

The invention is based in part on the discovery of fatty acid amides and their demonstrated effects in achieving improved treatment that cannot be achieved by administering the individual components (i.e. fatty acid and amine) or in noncovalent combination. Fatty acid amides and compositions comprising them are useful in the treatment and prevention of diseases and disorders associated with inflammation. For example, the fatty acid amides described herein are useful in the treatment of autoimmune diseases such as rheumatoid arthritis, psoriasis, systemic lupus erythematosus, inflammatory bowel diseases (including colitis and Crohn's disease), respiratory diseases such as asthma, cystic fibrosis, COPD and neurodegenerative diseases such as multiple sclerosis, Huntington's disease, Parkinson's disease and Alzheimer's disease, Huntington's disease, amyotrophic lateral sclerosis (ALS) and muscular dystrophy.

In one aspect, compounds of the **Formula I** are described: 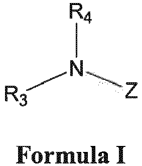 and pharmaceutically acceptable salts, enantiomers and stereoisomers thereof;
wherein
Z is 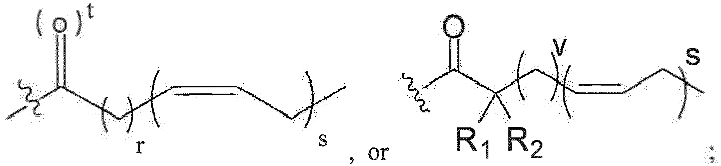
each r is independently 2, 3, or 7;
each s is independently 3, 5, or 6;
each t is independently 0 or 1;
each v is independently 1, 2, or 6;
R₁ and R₂ are independently -H, -D, -C₁-C₄ alkyl, -halogen, -OH, -C(O)C₁-C₄ alkyl, -O-aryl, -O-benzyl, -OC(O)C₁-C₄ alkyl, -C₂-C₃ alkene, -C₂-C₃ alkyne, -C(O)C₁-C₄ alkyl, -NH₂, -NH(C₁-C₃ alkyl), -N(C₁-C₃ alkyl)₂, -NH(C(O)C₁-C₃ alkyl), -N(C(O)C₁-C₃ alkyl)₂, -SH, -S(C₁-C₃ alkyl), -S(O)C₁-C₃ alkyl, -S(O)₂C₁-C₃ alkyl;
R₃ is H; and
R₄ is selected from: 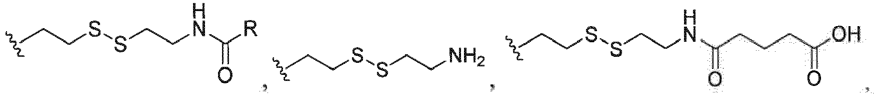 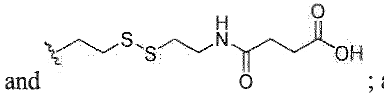 and
R is straight or branched -C₁-C₆ alkyl.

In another aspect, the compounds 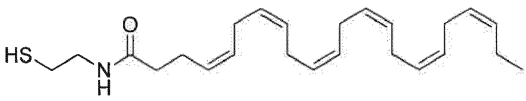 and 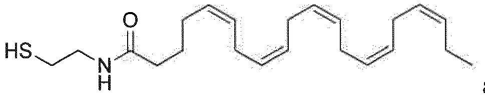 are described.

In **Formula I,** any one or more of H may be substituted with a deuterium.

Also described herein are compounds of the invention for use in methods of treating autoimmune disease, respiratory disease, or neurodegenerative diseases by administering to a patient in need thereof an effective amount of a compound of the invention.

The invention also includes pharmaceutical compositions that comprise an effective amount of a fatty acid amide and a pharmaceutically acceptable carrier. The compositions are useful for treating or preventing an autoimmune disease, respiratory disease, or neurodegenerative diseases. The invention includes a fatty acid amide provided as a pharmaceutically acceptable salt, enantiomer, stereoisomer, or mixtures thereof.

The details of the invention are set forth in the accompanying description below. Although methods and materials similar or equivalent to those described herein can be used in the practice or testing of the present invention, illustrative methods and materials are now described. Other features, objects, and advantages of the invention will be apparent from the description and from the claims. Unless defined otherwise, all technical and scientific terms used herein have the same meaning as commonly understood by one of ordinary skill in the art to which this invention belongs.

### DETAILED DESCRIPTION OF THE INVENTION

The invention is defined by the appended claims.

Metabolic disorders are a wide variety of medical disorders that interfere with a subject's metabolism. Metabolism is the process a subject's body uses to transform food into energy. Metabolism in a subject with a metabolic disorder is disrupted in some way. Autoimmune diseases arise from an overactive immune response of the body against tissues normally present in the body. Neurodegenerative diseases result from the deterioration of neurons or their myelin sheaths, which would eventually lead to a variety of CNS-related dysfunctions. The fatty acid amides possess the ability to treat or prevent autoimmune, respiratory diseases, or neurodegenerative diseases.

### DEFINITIONS

The following definitions are used in connection with the fatty acid amides:

The term "and/or" is used in this disclosure to mean either "and" or "or" unless indicated otherwise.

Unless otherwise specifically defined, the term "aryl" refers to cyclic, aromatic hydrocarbon groups that have 1 to 2 aromatic rings, including monocyclic or bicyclic groups such as phenyl, biphenyl or naphthyl. Where containing two aromatic rings (bicyclic, *etc*.), the aromatic rings of the aryl group may be joined at a single point (*e.g.,* biphenyl), or fused (*e.g.,* naphthyl). The aryl group may be optionally substituted by one or more substituents, *e.g.,* 1 to 5 substituents, at any point of attachment. The substituents can themselves be optionally substituted. It is understood that any of the substitutable hydrogens on an aryl can be substituted with halogen, C₁-C₃ alkyl, hydroxyl, alkoxy and cyano groups.

"C₁-C₃ alkyl" refers to a straight or branched chain saturated hydrocarbon containing 1-3 carbon atoms. Examples of a C₁-C₃ alkyl group include, methyl, ethyl, propyl and isopropyl.

"C₁-C₄ alkyl" refers to a straight or branched chain saturated hydrocarbon containing 1-4 carbon atoms. Examples of a C₁-C₄ alkyl group include, methyl, ethyl, propyl, butyl, isopropyl, isobutyl, *sec*-butyl and *tert*-butyl.

"C₁-C₅ alkyl" refers to a straight or branched chain saturated hydrocarbon containing 1-5 carbon atoms. Examples of a C₁-C₅ alkyl group include, methyl, ethyl, propyl, butyl, pentyl, isopropyl, isobutyl, *sec*-butyl and *tert*-butyl, isopentyl and neopentyl.

"C₁-C₆ alkyl" refers to a straight or branched chain saturated hydrocarbon containing 1-6 carbon atoms. Examples of a C₁-C₆ alkyl group include, methyl, ethyl, propyl, butyl, pentyl, hexyl, isopropyl, isobutyl, *sec*-butyl, *tert*-butyl, isopentyl, and neopentyl. It is understood that any of the substitutable hydrogens on a C₁-C₆ alkyl can be substituted with halogen, hydroxyl, alkoxy and cyano groups.

The term "cycloalkyl" refers to a cyclic hydrocarbon containing 3-6 carbon atoms. Examples of a cycloalkyl group include, cyclopropyl, cyclobutyl, cyclopentyl and cyclohexyl. It is understood that any of the substitutable hydrogens on a cycloalkyl can be substituted with halogen, C₁-C₃ alkyl, hydroxyl, alkoxy and cyano groups.

The term "heterocycle" as used herein refers to a cyclic hydrocarbon containing 3-6 atoms wherein at least one of the atoms is an O, N, or S. Examples of heterocycles include, aziridine, oxirane, thiirane, azetidine, oxetane, thietane, pyrrolidine, tetrahydrofuran, tetrahydrothiophene, piperidine, tetrahydropyran, thiane, imidazolidine, oxazolidine, thiazolidine, dioxolane, dithiolane, piperazine, oxazine, dithiane and dioxane. It is understood that any of the substitutable hydrogens on a heterocycle can be substituted with halogen, C₁-C₃ alkyl, hydroxyl, alkoxy and cyano groups.

The term "heteroaryl" as used herein refers to a monocyclic or bicyclic ring structure having 5 to 12 ring atoms wherein one or more of the ring atoms is a heteroatom, e.g. N, O or S and wherein one or more rings of the bicyclic ring structure is aromatic. Some examples of heteroaryl are pyridyl, furyl, pyrrolyl, thienyl, thiazolyl, oxazolyl, imidazolyl, indolyl, tetrazolyl, benzofuryl, xanthenes and dihydroindole. It is understood that any of the substitutable hydrogens on a heteroaryl can be substituted with halogen, C₁-C₃ alkyl, hydroxyl, alkoxy and cyano groups.

The term "any one of the side chains of the naturally occurring amino acids" as used herein means a side chain of any one of the following amino acids: Isoleucine, Alanine, Leucine, Asparagine, Lysine, Aspartate, Methionine, Cysteine, Phenylalanine, Glutamate, Threonine, Glutamine, Tryptophan, Glycine, Valine, Proline, Arginine, Serine, Histidine and Tyrosine.

The term "fatty acid" as used herein means an omega-3 fatty acid and fatty acids that are metabolized *in vivo* to omega-3 fatty acids. Examples of fatty acids are *all-cis*-7,10,13-hexadecatrienoic acid, α-linolenic acid (ALA or *all-cis*-9,12,15-octadecatrienoic acid), stearidonic acid (STD or *all-cis*-6,9,12,15-octadecatetraenoic acid), eicosatrienoic acid (ETE or *all-cis*-11,14,17-eicosatrienoic acid), eicosatetraenoic acid (ETA or *all-cis*-8,11,14,17-eicosatetraenoic acid), eicosapentaenoic acid (EPA or *all-cis-*5,8,11,14,17-eicosapentaenoic acid), docosapentaenoic acid (DPA, clupanodonic acid or *all-cis-7,10,13,16,19-docosapentaenoic* acid), docosahexaenoic acid (DHA or *all-cis-*4,7,10,13,16,19-docosahexaenoic acid), tetracosapentaenoic acid (*all-cis*-9,12,15,18,21-docosahexaenoic acid), or tetracosahexaenoic acid (nisinic acid or *all-cis*-6,9,12,15,18,21-tetracosenoic acid).

A "subject" is a mammal, *e.g.,* a human, mouse, rat, guinea pig, dog, cat, horse, cow, pig, or non-human primate, such as a monkey, chimpanzee, baboon or rhesus, and the terms "subject" and "patient" are used interchangeably herein.

The invention also includes pharmaceutical compositions comprising an effective amount of a fatty acid amide and a pharmaceutically acceptable carrier. The invention includes a fatty acid amide provided as a pharmaceutically acceptable salt, enantiomers, stereoisomers, or mixtures thereof.

Representative "pharmaceutically acceptable salts" include, *e.g.,* water-soluble and water-insoluble salts, such as the acetate, amsonate (4,4-diaminostilbene-2, 2 - disulfonate), benzenesulfonate, benzonate, bicarbonate, bisulfate, bitartrate, borate, bromide, butyrate, calcium, calcium edetate, camsylate, carbonate, chloride, citrate, clavulariate, dihydrochloride, edetate, edisylate, estolate, esylate, fiunarate, gluceptate, gluconate, glutamate, glycollylarsanilate, hexafluorophosphate, hexylresorcinate, hydrabamine, hydrobromide, hydrochloride, hydroxynaphthoate, iodide, isothionate, lactate, lactobionate, laurate, magnesium, malate, maleate, mandelate, mesylate, methylbromide, methylnitrate, methylsulfate, mucate, napsylate, nitrate, *N*-methylglucamine ammonium salt, 3-hydroxy-2-naphthoate, oleate, oxalate, palmitate, pamoate (1,1-methene-bis-2-hydroxy-3-naphthoate, einbonate), pantothenate, phosphate/diphosphate, picrate, polygalacturonate, propionate, p-toluenesulfonate, salicylate, stearate, subacetate, succinate, sulfate, sulfosalicylate, suramate, tannate, tartrate, teoclate, tosylate, triethiodide, and valerate salts.

The term "metabolic disease" as used herein refers to disorders, diseases and syndromes involving dyslipidemia, and the terms metabolic disorder, metabolic disease, and metabolic syndrome are used interchangeably herein.

An "effective amount" when used in connection with a fatty acid amide is an amount effective for treating or preventing a metabolic disease.

The term "carrier", as used in this disclosure, encompasses carriers, excipients, and diluents and means a material, composition or vehicle, such as a liquid or solid filler, diluent, excipient, solvent or encapsulating material, involved in carrying or transporting a pharmaceutical agent from one organ, or portion of the body, to another organ, or portion of the body.

The term "treating", with regard to a subject, refers to improving at least one symptom of the subject's disorder. Treating can be curing, improving, or at least partially ameliorating the disorder.

The term "disorder" is used in this disclosure to mean, and is used interchangeably with, the terms disease, condition, or illness, unless otherwise indicated.

The term "administer", "administering", or "administration" as used in this disclosure refers to either directly administering a compound or pharmaceutically acceptable salt of the compound or a composition to a subject, or
pharmaceutically acceptable salt of the compound or composition to the subject, which can form an equivalent amount of active compound within the subject's body.

The following abbreviations are used herein and have the indicated definitions: Boc and BOC are *tert*-butoxycarbonyl, Boc₂O is di-*tert*-butyl dicarbonate, CDI is 1,1'-carbonyldiimidazole, DCC is *N,N*'-dicyclohexylcarbodiimide, DIEA is *N,N-*diisopropylethylamine, DMAP is 4-dimethylaminopyridine, DOSS is sodium dioctyl sulfosuccinate, EDC and EDCI are 1-ethyl-3-(3-dimethylaminopropyl)carbodiimide hydrochloride, EtOAc is ethyl acetate, h is hour, HATU is 2-(7-aza-1*H* benzotriazole-1-yl)-1,1,3,3-tetramethyluronium hexafluorophosphate, HPMC is hydroxypropyl methylcellulose, oxone is potassium peroxymonosulfate, Pd/C is palladium on carbon, TFA is trifluoroacetic acid, TGPS is tocopherol propylene glycol succinate, THF is tetrahydrofuran, and TNF is tumor necrosis factor.

### COMPOUNDS

Accordingly in one aspect, a fatty acid amide according to **Formula I** is.

In some embodiments, the fatty acids are selected from the group consisting of *all-cis*-7,10,13-hexadecatrienoic acid, α-linolenic acid, stearidonic acid, eicosatrienoic acid, eicosatetraenoic acid, eicosapentaenoic acid (EPA), docosapentaenoic acid, docosahexaenoic acid (DHA), tetracosapentaenoic acid, tetracosahexaenoic acid, and lipoic acid. In other embodiments, the fatty acid is selected from eicosapentaenoic acid,docosahexaenoic acid, and lipoic acid. In other embodiments, the fatty acid is selected from eicosapentaenoic acid and docosahexaenoic acid. In some embodiments, the hydrolysis is enzymatic.

In another aspect, the present invention provides fatty acid amides according to **Formula I:** 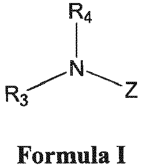 and pharmaceutically acceptable salts, enantiomers and stereoisomers thereof;
wherein m, e, r, s, t, v, z, R₁, R₂, R₃, R₄, are as defined above for **Formula I,**

In some embodiments, Z is 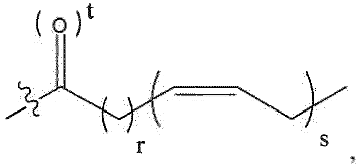 and r is 2.

In some embodiments, Z is 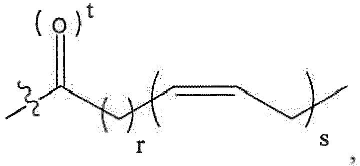 and r is 3.

In some embodiments, Z is 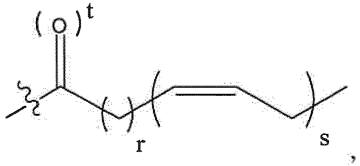 and r is 7.

In other embodiments, Z is 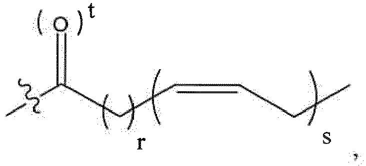 and s is 3.

In some embodiments, Z is 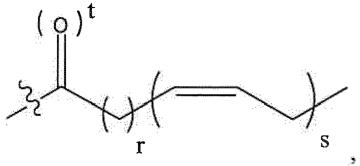 and s is 5.

In some embodiments, Z is 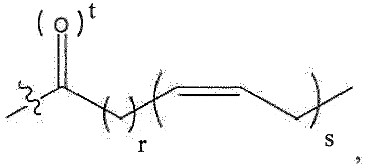 and s is 6.

In some embodiments, Z is 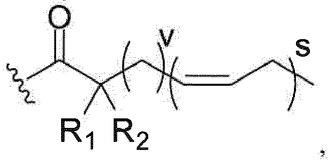 and v is 1.

In other embodiments, Z is 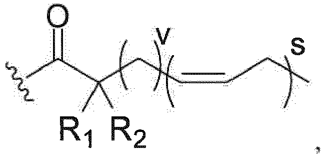 and v is 2.

In some embodiments, Z is 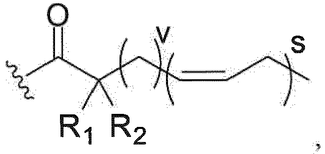 and v is 6.

In some embodiments, Z is 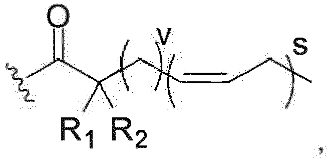 and s is 3.

In some embodiments, Z is 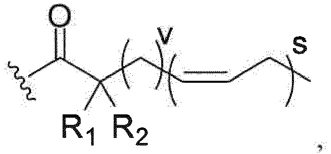 and s is 5.

In other embodiments, Z is 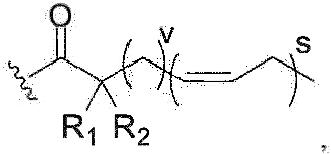 and s is 6.

In some embodiments, r is 2 and s is 6.

In some embodiments, r is 3 and s is 5.

In some embodiments, t is 1.

In some embodiments, R₃ is H and R₄ is

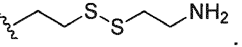

In some embodiments, R₃ is H and R₄ is

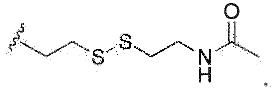

In some embodiments, R₃ is H and R₄ is

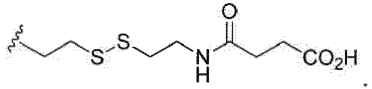

In some embodiments, R₃ is H and R₄ is

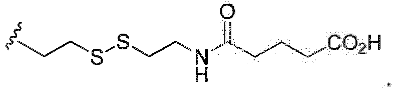

In **Formula I,** any one or more of H may be substituted with a deuterium.

Disclosed are furthermore the following compounds: 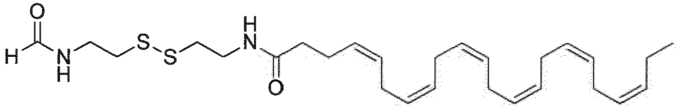 (4Z,7Z,10Z,13Z,16Z,19Z)-N-(2-((2-formamidoethyl)disulfanyl)ethyl)docosa-4,7,10,13,16,19-hexaenamide (**I-1**); 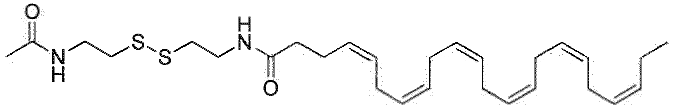 (4Z,7Z,10Z,13Z,16Z,19Z)-N-(2-((2-acetamidoethyl)disulfanyl)ethyl)docosa-4,7,10,13,16,19-hexaenamide **(1-2);** 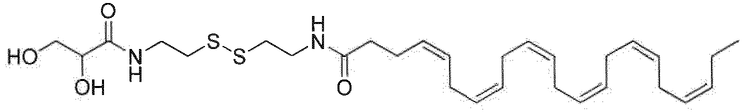 (4Z,7Z,10Z,13Z,16Z,19Z)-N-(2-((2-(2,3-dihydroxypropanamido)ethyl)disulfanyl)ethyl)docosa-4,7,10,13,16,19-hexaenamide **(I-3);** 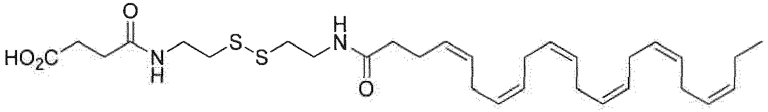 4-((2-((2-((4Z,7Z,10Z,13Z,16Z, 19Z)-docosa-4,7,10,13,16,19-hexaenamido)ethyl)disulfanyl)ethyl)amino)-4-oxobutanoic acid **(I-4);** 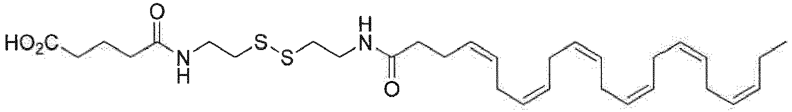 5-((2-((2-((4Z,7Z,10Z,13Z, 16Z,19Z)-docosa-4,7,10,13,16,19-hexaenamido)ethyl)disulfanyl)ethyl)amino)-5-oxopentanoic acid **(I-5);** 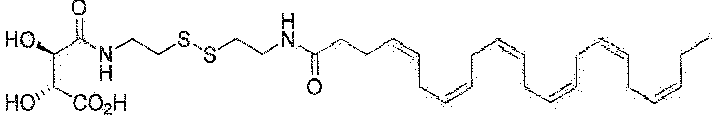 (2R,3R)-4-((2-((2-((4Z,7Z,10Z, 13Z,16Z,19Z)-docosa-4,7,10,13,16,19-hexaenamido)ethyl)disulfanyl)ethyl)amino)-2,3-dihydroxy-4-oxobutanoic acid **(I-6);** 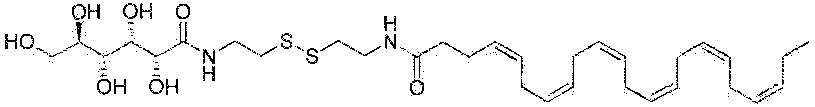 (4Z,7Z,10Z,13Z,16Z,19Z)-N-(2-((2-((2R,3S,4R,5R)-2,3,4,5,6-pentahydroxyhexanamido)ethyl)disulfanyl)ethyl)docosa-4,7,10,13,16,19-hexaenamide **(I-7);** 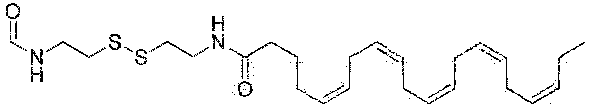 (5Z,8Z,11Z,14Z,17Z)-N-(2-((2-formamidoethyl)disulfanyl)ethyl)icosa-5,8,11,14,17-pentaenamide **(I-8);** 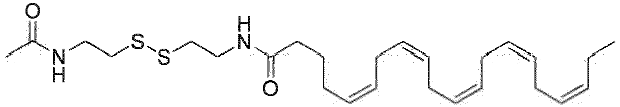 (5Z,8Z,11Z,14Z,17Z)-N-(2-((2-acetamidoethyl)disulfanyl)ethyl)icosa-5,8,11,14,17-pentaenamide **(I-9);** 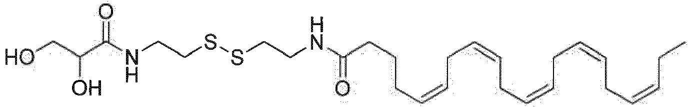 (5Z,8Z,11Z,14Z,17Z)-N-(2-((2-(2,3-dihydroxypropanamido)ethyl)disulfanyl)ethyl)icosa-5,8,11,14,17-pentaenamide (**I-10**); 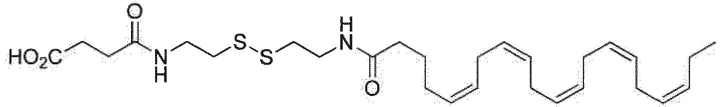 4-((2-((2-((5Z,8Z,11Z,14Z,17Z)-icosa-5,8,11,14,17-pentaenamido)ethyl)disulfanyl)ethyl)amino)-4-oxobutanoic acid **(I-11);** 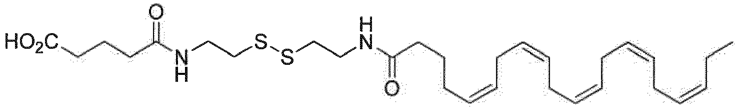 5-((2-((2-((5Z,8Z,11Z,14Z,17Z)-icosa-5,8,11,14,17-pentaenamido)ethyl)disulfanyl)ethyl)amino)-5-oxopentanoic acid **(I-12);** 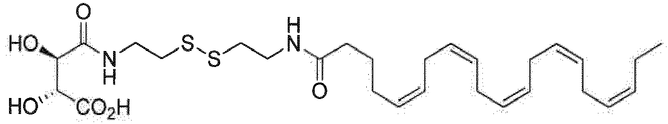 (2R,3R)-2,3-dihydroxy-4-((2-((2-((5Z,8Z,11Z,14Z,17Z)-icosa-5,8,11,14,17-pentaenamido)ethyl)disulfanyl)ethyl)amino)-4-oxobutanoic acid **(I-13);** 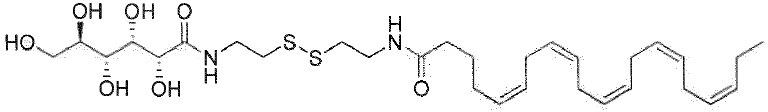 (5Z,8Z,11Z,14Z,17Z)-N-(2-((2-((2R,3 S,4R,5R)-2,3,4,5,6-pentahydroxyhexanamido)ethyl)disulfanyl)ethyl)icosa-5,8,11,14,17-pentaenamide **(I-14);** 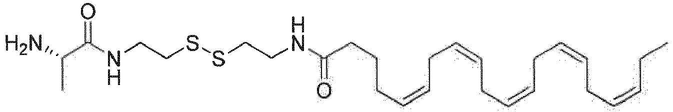 (5Z,8Z,11Z,14Z,17Z)-N-(2-((2-((S)-2-aminopropanamido)ethyl)disulfanyl)ethyl)icosa-5,8,11,14,17-pentaenamide (**I-15**); 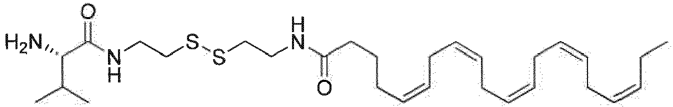 (5Z,8Z,11Z,14Z,17Z)-N-(2-((2-((S)-2-amino-3-methylbutanamido)ethyl)disulfanyl)ethyl)icosa-5,8,11,14,17-pentaenamide **(I-16);** 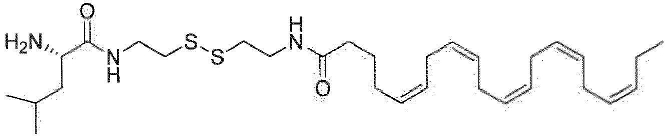 (5Z,8Z,11Z,14Z,17Z)-N-(2-((2-((S)-2-amino-4-methylpentanamido)ethyl)disulfanyl)ethyl)icosa-5,8,11,14,17-pentaenamide **(I-17)**; 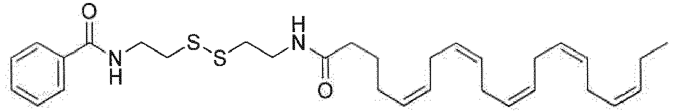 N-(2-((2-((5Z,8Z,11Z,14Z,17Z)-icosa-5,8,11,14,17-pentaenamido)ethyl)disulfanyl)ethyl)benzamide **(I-18)**; 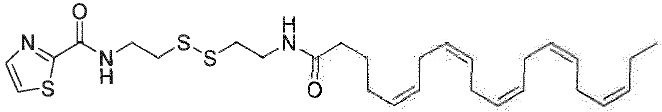 N-(2-((2-((5Z,8Z,11Z,14Z,17Z)-icosa-5,8,11,14,17-pentaenamido)ethyl)disulfanyl)ethyl)thiazole-2-carboxamide **(I-19);** 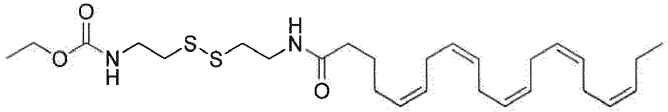 ethyl (2-((2-((5Z,8Z,11Z,14Z,17Z)-icosa-5,8,11,14,17-pentaenamido)ethyl)disulfanyl)ethyl)carbamate **(I-20);** 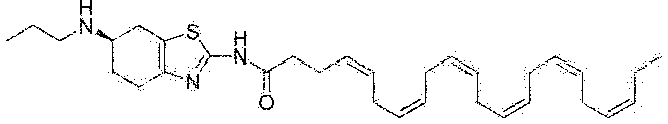 (4Z,7Z,10Z,13Z,16Z,19Z)-N-((R)-6-(propylamino)-4,5,6,7-tetrahydrobenzo[d]thiazol-2-yl)docosa-4,7,10,13,16,19-hexaenamide **(I-21)**; 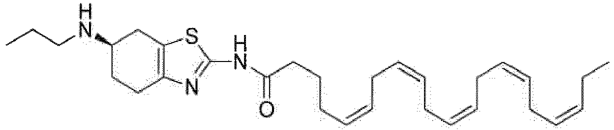 (5Z,8Z,11Z,14Z,17Z)-N-((R)-6-(propylamino)-4,5,6,7-tetrahydrobenzo[d]thiazol-2-yl)icosa-5,8,11,14,17-pentaenamide **(I-22)**; 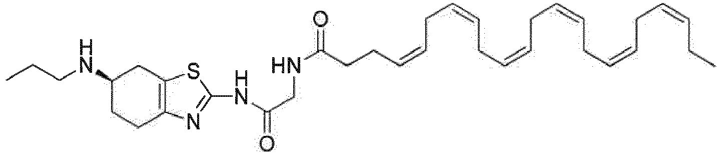 (4Z,7Z,10Z,13Z,16Z,19Z)-N-(2-oxo-2-(((R)-6-(propylamino)-4,5,6,7-tetrahydrobenzo[d]thiazol-2-yl)amino)ethyl)docosa-4,7,10,13,16,19-hexaenamide **(I-23)**; 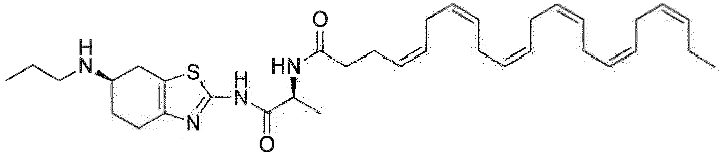 (4Z,7Z,10Z,13Z,16Z,19Z)-N-((S)-1-oxo-1-(((R)-6-(propylamino)-4,5,6,7-tetrahydrobenzo[d]thiazol-2-yl)amino)propan-2-yl)docosa-4,7,10,13,16,19-hexaenamide **(I-24);** 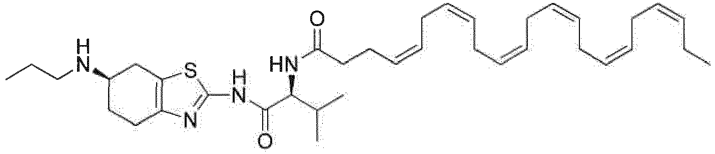 (4Z,7Z,10Z,13Z,16Z,19Z)-N-((S)-3-methyl-1-oxo-1-(((R)-6-(propylamino)-4,5,6,7-tetrahydrobenzo[d]thiazol-2-yl)amino)butan-2-yl)docosa-4,7,10,13,16,19-hexaenamide **(I-25);** 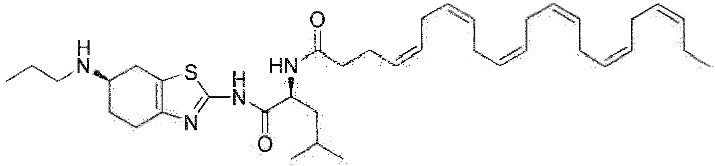 (4Z,7Z,10Z,13Z,16Z,19Z)-N-((S)-4-methyl-1-oxo-1-(((R)-6-(propylamino)-4,5,6,7-tetrahydrobenzo[d]thiazol-2-yl)amino)pentan-2-yl)docosa-4,7,10,13,16,19-hexaenamide **(I-26);** 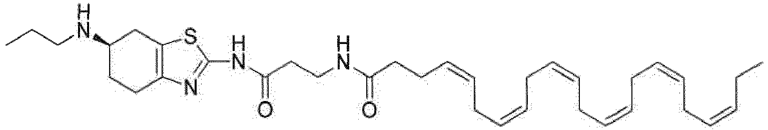 (4Z,7Z,10Z,13Z,16Z,19Z)-N-(3-oxo-3-(((R)-6-(propylamino)-4,5,6,7-tetrahydrobenzo[d]thiazol-2-yl)amino)propyl)docosa-4,7,10,13,16,19-hexaenamide **(I-27);** 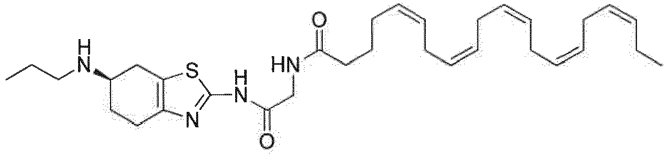 (5Z,8Z,11Z,14Z,17Z)-N-(2-oxo-2-(((R)-6-(propylammo)-4,5,6,7-tetrahydrobenzo[d]thiazol-2-yl)amino)ethyl)icosa-5,8,11,14,17-pentaenamide **(I-28);** 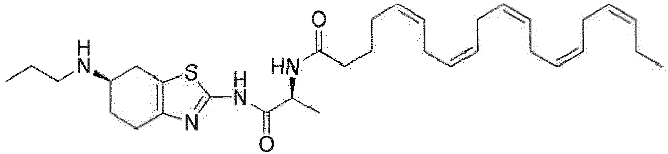 (5Z,8Z,11Z,14Z,17Z)-N-((S)-1-oxo-1-(((R)-6-(propylamino)-4,5,6,7-tetrahydrobenzo[d]thiazol-2-yl)amino)propan-2-yl)icosa-5,8,11,14,17-pentaenamide **(I-29);** 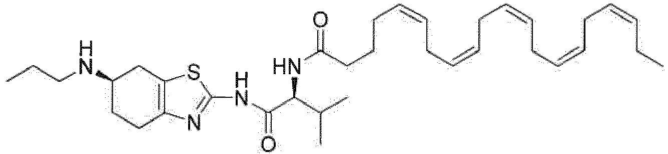 (5Z,8Z,11Z,14Z,17Z)-N-((S)-3-methyl-1-oxo-1-(((R)-6-(propylamino)-4,5,6,7-tetrahydrobenzo[d]thiazol-2-yl)amino)butan-2-yl)icosa-5,8,11,14,17-pentaenamide **(I-30);** 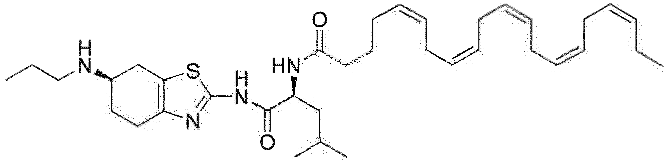 (5Z,8Z,11Z,14Z,17Z)-N-((S)-4-methyl-1-oxo-1-(((R)-6-(propylamino)-4,5,6,7-tetrahydrobenzo[d]thiazol-2-yl)amino)pentan-2-yl)icosa-5,8,11,14,17-pentaenamide (**I-31**); 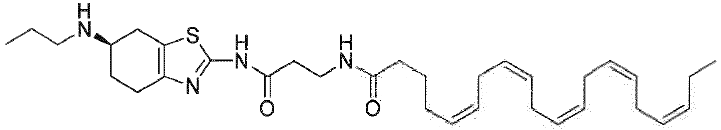 (5Z,8Z,11Z,14Z,17Z)-N-(3-oxo-3-(((R)-6-(propylamino)-4,5,6,7-tetrahydrobenzo[d]thiazol-2-yl)amino)propyl)icosa-5,8,11,14,17-pentaenamide **(I-32);** 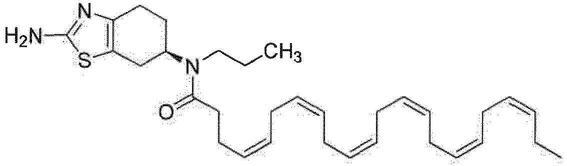 (4Z,7Z,10Z,13Z,16Z,19Z)-N-((R)-2-amino-4,5,6,7-tetrahydrobenzo[d]thiazol-6-yl)-N-propyldocosa-4,7,10,13,16,19-hexaenamide **(I-33);** 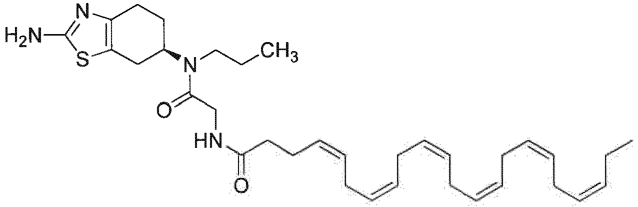 (4Z,7Z, 10Z, 13Z, 16Z, 19Z)-N-(2-(((R)-2-amino-4,5,6,7-tetrahydrobenzo[d]thiazol-6-yl)(propyl)amino)-2-oxoethyl)docosa-4,7,10,13,16,19-hexaenamide **(I-34);** 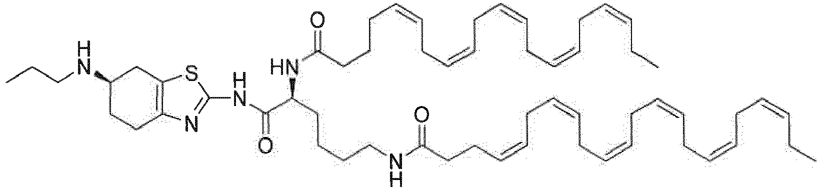 (4Z,7Z,10Z,13Z,16Z,19Z)-N-((S)-5-((5Z,8Z,11Z,14Z,17Z)-icosa-5,8,11,14,17-pentaenamido)-6-oxo-6-(((R)-6-(propylamino)-4,5,6,7-tetrahydrobenzo[d]thiazol-2-yl)amino)hexyl)docosa-4,7,10,13,16,19-hexaenamide **(I-35);** 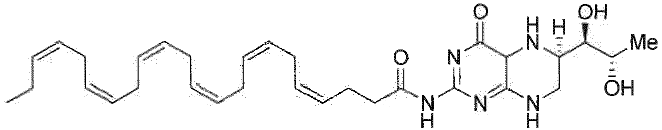 (4Z,7Z,10Z,13Z,16Z,19Z)-N-((6R)-6-((1R,2S)-1,2-dihydroxypropyl)-4-oxo-4,4a,5,6,7,8-hexahydropteridin-2-yl)docosa-4,7,10,13,16,19-hexaenamide **(I-36);** 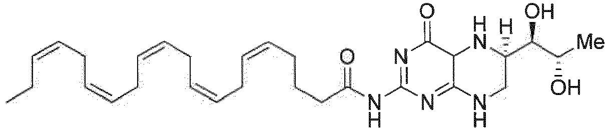 (5Z,8Z,11Z,14Z,17Z)-N-((6R)-6-((1R,2S)-1,2-dihydroxypropyl)-4-oxo-4,4a,5,6,7,8-hexahydropteridin-2-yl)icosa-5,8,11,14,17-pentaenamide **(I-37);** 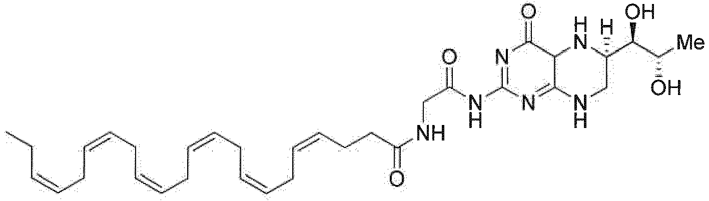 (4Z,7Z,10Z,13Z,16Z,19Z)-N-(2-(((6R)-6-((1R,2S)-1,2-dihydroxypropyl)-4-oxo-4,4a,5,6,7,8-hexahydropteridin-2-yl)amino)-2-oxoethyl)docosa-4,7,10,13,16,19-hexaenamide **(I-38);** 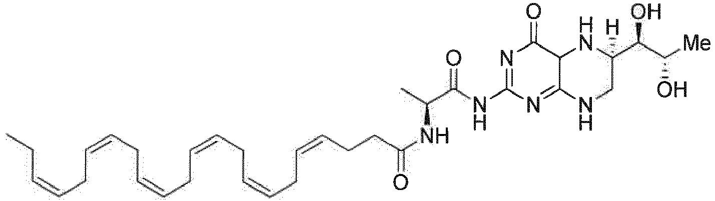 (4Z,7Z,10Z,13Z,16Z,19Z)-N-((2S)-1-(((6R)-6-((1R,2S)-1,2-dihydroxypropyl)-4-oxo-4,4a,5,6,7,8-hexahydropteridin-2-yl)amino)-1-oxopropan-2-yl)docosa-4,7,10,13,16,19-hexaenamide **(I-39);** 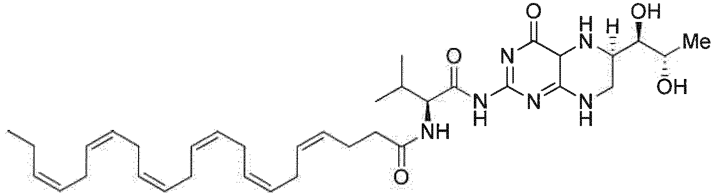 (4Z,7Z,10Z,13Z,16Z,19Z)-N-((2S)-1-(((6R)-6-((1R,2S)-1,2-dihydroxypropyl)-4-oxo-4,4a,5,6,7,8-hexahydropteridin-2-yl)amino)-3-methyl-1-oxobutan-2-yl)docosa-4,7,10,13,16,19-hexaenamide **(I-40);** 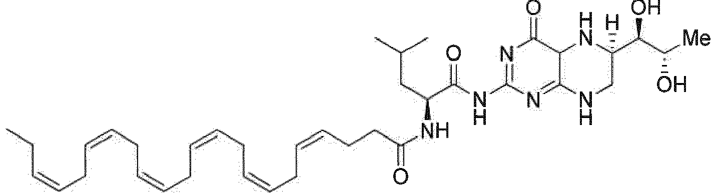 (4Z,7Z,10Z,13Z,16Z,19Z)-N-((2S)-1-(((6R)-6-((1R,2S)-1,2-dihydroxypropyl)-4-oxo-4,4a,5,6,7,8-hexahydropteridin-2-yl)amino)-4-methyl-1-oxopentan-2-yl)docosa-4,7,10,13,16,19-hexaenamide **(I-41);** 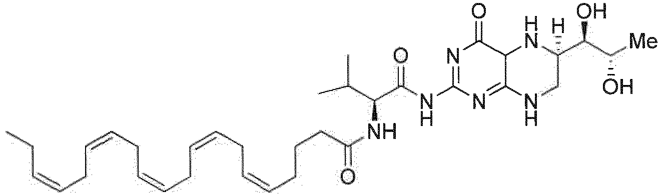 (5Z,8Z,11Z,14Z,17Z)-N-((2S)-1-(((6R)-6-((1R,2S)-1,2-dihydroxypropyl)-4-oxo-4,4a,5,6,7,8-hexahydropteridin-2-yl)amino)-3-methyl-1-oxobutan-2-yl)icosa-5,8,11,14,17-pentaenamide **(I-42);** 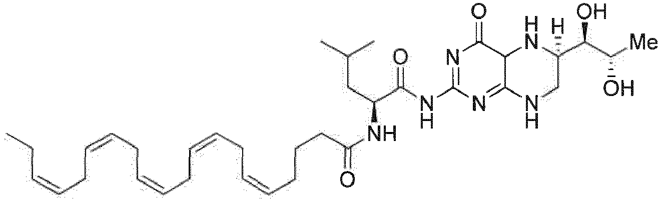 (5Z,8Z,11Z,14Z,17Z)-N-((2S)-1-(((6R)-6-((1R,2S)-1,2-dihydroxypropyl)-4-oxo-4,4a,5,6,7,8-hexahydropteridin-2-yl)amino)-4-methyl-1-oxopentan-2-yl)icosa-5,8,11,14,17-pentaenamide **(I-43);** 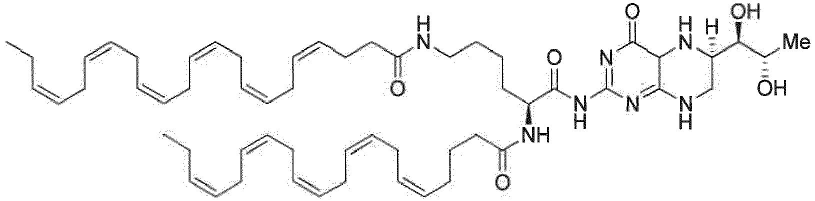 (4Z,7Z,10Z,13Z,16Z,19Z)-N-((5S)-6-(((6R)-6-((1R,2S)-1,2-dihydroxypropyl)-4-oxo-4,4a,5,6,7,8-hexahydropteridin-2-yl)amino)-5-((5Z,8Z,11Z,14Z,17Z)-icosa-5,8,11,14,17-pentaenamido)-6-oxohexyl)docosa-4,7,10,13,16,19-hexaenamide **(I-44);** 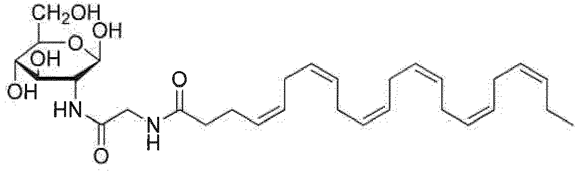 (4Z,7Z,10Z,13Z,16Z,19Z)-N-(2-oxo-2-(((2R,3R,4R,5S,6R)-2,4,5-trihydroxy-6-(hydroxymethyl)tetrahydro-2H-pyran-3-yl)amino)ethyl)docosa-4,7,10,13,16,19-hexaenamide **(I-45);** 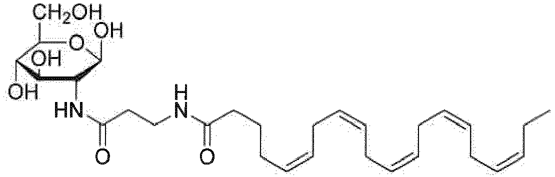 (5Z,8Z,11Z,14Z,17Z)-N-(3-oxo-3-(((2R,3R,4R,5S,6R)-2,4,5-trihydroxy-6-(hydroxymethyl)tetrahydro-2H-pyran-3-yl)amino)propyl)icosa-5,8,11,14,17-pentaenamide **(I-46);** 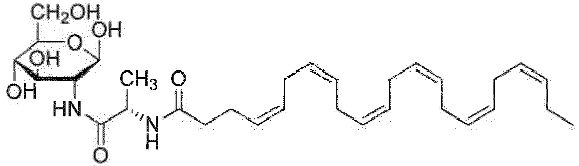 (4Z,7Z,10Z,13Z,16Z,19Z)-N-((S)-1-oxo-1-(((2R,3R,4R,5S,6R)-2,4,5-trihydroxy-6-(hydroxymethyl)tetrahydro-2H-pyran-3-yl)amino)propan-2-yl)docosa-4,7,10,13,16,19-hexaenamide **(I-47);** 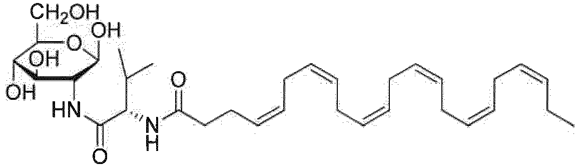 (4Z,7Z,10Z,13Z,16Z,19Z)-N-((S)-3-methyl-1-oxo-1-(((2R,3R,4R,5S,6R)-2,4,5-trihydroxy-6-(hydroxymethyl)tetrahydro-2H-pyran-3-yl)amino)butan-2-yl)docosa-4,7,10,13,16,19-hexaenamide **(I-48);** 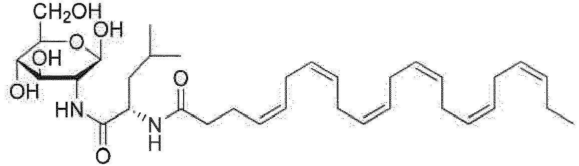 (4Z,7Z,10Z,13Z,16Z,19Z)-N-((S)-4-methyl-1-oxo-1-(((2R,3R,4R,5S,6R)-2,4,5-trihydroxy-6-(hydroxymethyl)tetrahydro-2H-pyran-3-yl)amino)pentan-2-yl)docosa-4,7,10,13,16,19-hexaenamide **(I-49);** 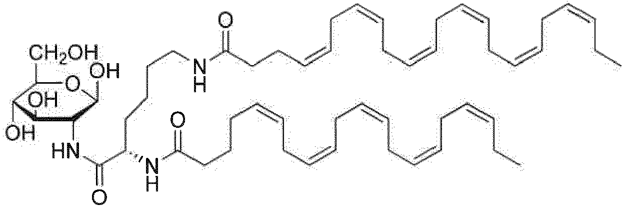 (4Z,7Z,10Z,13Z,16Z,19Z)-N-((S)-5-((5Z,8Z,11Z,14Z,17Z)-icosa-5,8,11,14,17-pentaenamido)-6-oxo-6-(((2R,3R,4R,5S,6R)-2,4,5-trihydroxy-6-(hydroxymethyl)tetrahydro-2H-pyran-3-yl)amino)hexyl)docosa-4,7,10,13,16,19-hexaenamide **(I-50);** 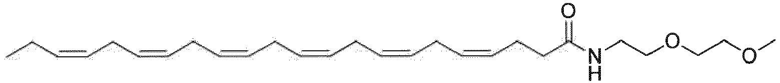 (4Z,7Z, 1 0Z,13Z, 16Z,19Z)-N-(2-(2-methoxyethoxy)ethyl)docosa-4,7,10,13,16,19-hexaenamide **(I-51);** 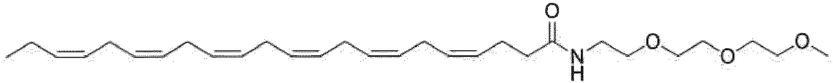 (4Z,7Z,10Z,13Z,16Z,19Z)-N-(2-(2-(2-methoxyethoxy)ethoxy)ethyl)docosa-4,7,10,13,16,19-hexaenamide **(I-52);** 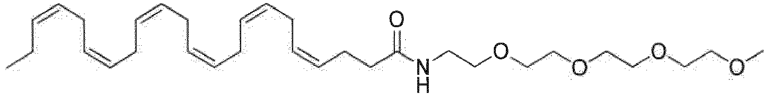 (4Z,7Z,10Z,13Z,16Z,19Z)-N-(2-(2-(2-(2-methoxyethoxy)ethoxy)ethoxy)ethyl)docosa-4,7,10,13,16,19-hexaenamide (**I-53**); 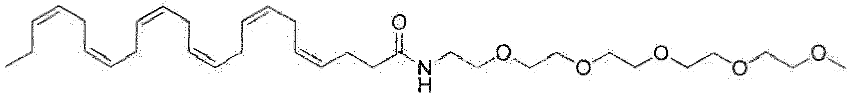 (4Z,7Z,10Z,13Z,16Z,19Z)-N-(2-(2-(2-(2-(2-methoxyethoxy)ethoxy)ethoxy)ethoxy)ethyl)docosa-4,7,10,13,16,19-hexaenamide (**I-54**); 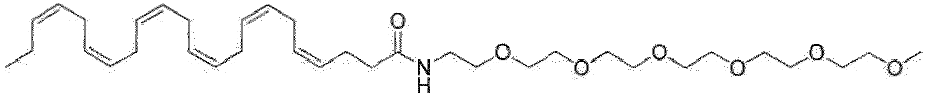 (4Z,7Z,10Z,13Z,16Z,19Z)-N-(2-(2-(2-(2-(2-(2-methoxyethoxy)ethoxy)ethoxy)ethoxy)ethoxy)ethyl)docosa-4,7,10,13,16,19-hexaenamide **(I-55);** 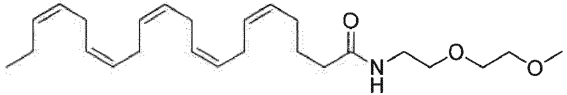 (5Z,8Z,11Z,14Z,17Z)-N-(2-(2-methoxyethoxy)ethyl)icosa-5,8,11,14,17-pentaenamide **(I-56);** 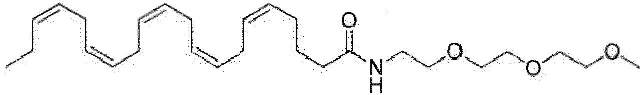 (5Z,8Z,11Z,14Z,17Z)-N-(2-(2-(2-methoxyethoxy)ethoxy)ethyl)icosa-5,8,11,14,17-pentaenamide **(I-57);** 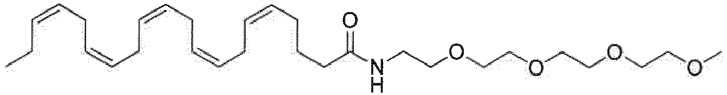 (5Z,8Z,11Z,14Z,17Z)-N-(2-(2-(2-(2-methoxyethoxy)ethoxy)ethoxy)ethyl)icosa-5,8,11,14,17-pentaenamide (**I-58**); 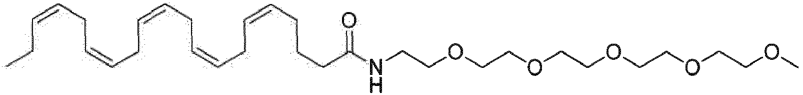 (5Z,8Z,11Z,14Z,17Z)-N-(2-(2-(2-(2-(2-methoxyethoxy)ethoxy)ethoxy)ethoxy)ethyl)icosa-5,8,11,14,17-pentaenamide (**I-59**); 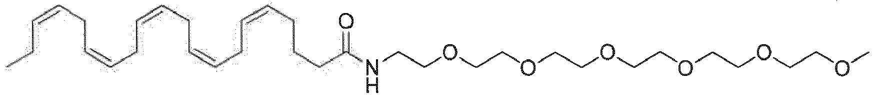 (5Z,8Z,11Z,14Z,17Z)-N-(2-(2-(2-(2-(2-(2-methoxyethoxy)ethoxy)ethoxy)ethoxy)ethoxy)ethyl)icosa-5,8,11,14,17-pentaenamide **(I-60).** 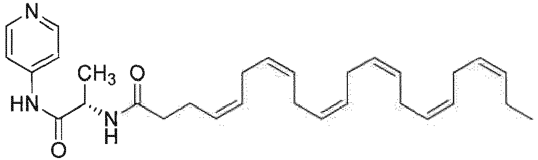 (4Z,7Z,10Z,13Z,16Z,19Z)-N-((S)-1-oxo-1-(pyridin-4-ylamino)propan-2-yl)docosa-4,7,10,13,16,19-hexaenamide **(I-61);** 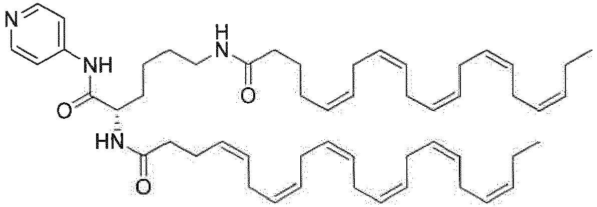 (4Z,7Z,10Z,13Z,16Z,19Z)-N-((S)-6-((5Z,8Z,11Z,14Z,17Z)-icosa-5,8,11,14,17-pentaenamido)-1-oxo-1-(pyridin-4-ylamino)hexan-2-yl)docosa-4,7,10,13,16,19-hexaenamide **(I-62);** 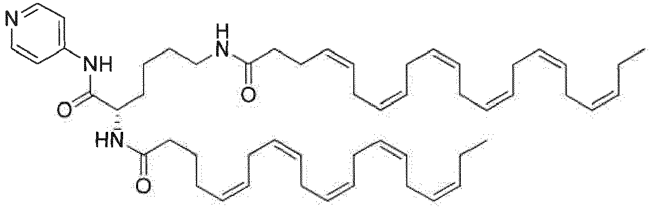 (4Z,7Z,10Z,13Z,16Z,19Z)-N-((S)-5-((5Z,8Z,11Z,14Z,17Z)-icosa-5,8,11,14,17-pentaenamido)-6-oxo-6-(pyridin-4-ylamino)hexyl)docosa-4,7,10,13,16,19-hexaenamide (**I-63);** 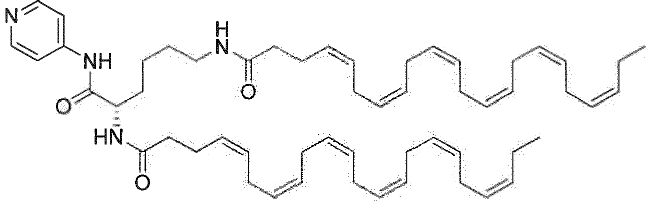 (4Z,4'Z,7Z,7'Z,10Z,10'Z,13Z,13'Z,16Z,16'Z,19Z,19'Z)-N,N'-((S)-6-oxo-6-(pyridin-4-ylamino)hexane-1,5-diyl)bis(docosa-4,7,10,13,16,19-hexaenamide) **(I-64);** 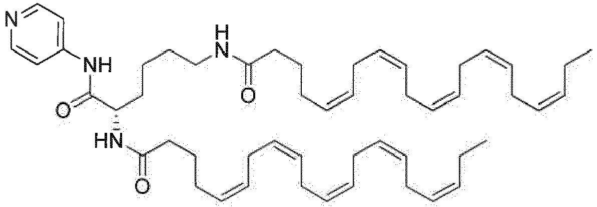 (5Z,5'Z,8Z,8'Z,11Z,11'Z,14Z,14'Z,17Z,17'Z)-N,N'-((S)-6-oxo-6-(pyridin-4-ylamino)hexane-1,5-diyl)bis(icosa-5,8,11,14,17-pentaenamide) **(I-65);** 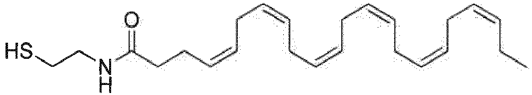 (4Z,7Z, 10Z,13Z,16Z,19Z)-N-(2-mercaptoethyl)docosa-4,7,10,13,16,19-hexaenamide **(I-66);** 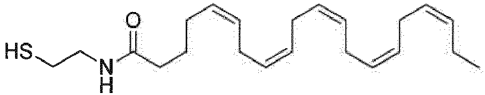 (5Z,8Z,11Z,14Z,17Z)-N-(2-mercaptoethyl)icosa-5,8,11,14,17-pentaenamide **(I-67)** 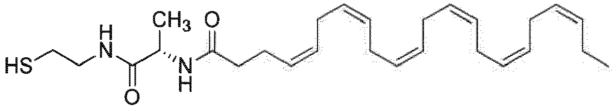 (4Z,7Z,10Z,13Z,16Z,19Z)-N-((S)-1-((2-mercaptoethyl)amino)-1-oxopropan-2-yl)docosa-4,7,10,13,16,19-hexaenamide **(I-68);** 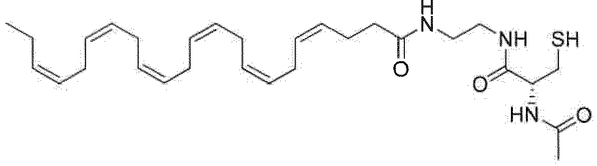 (4Z,7Z,10Z,13Z,16Z,19Z)-N-(2-((R)-2-acetamido-3-mercaptopropanamido)ethyl)docosa-4,7,10,13,16,19-hexaenamide **(I-69);** 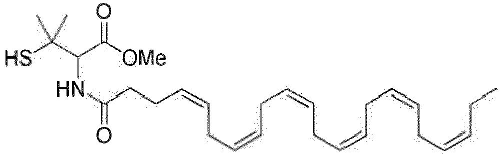 methyl 2-((4Z,7Z,10Z,13Z,16Z,19Z)-docosa-4,7,10,13,16,19-hexaenamido)-3-mercapto-3-methylbutanoate **(I-70);** 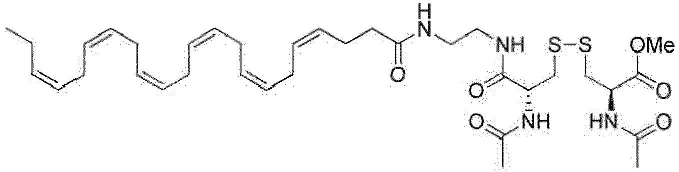 (R)-methyl 2-acetamido-3-(((R)-2-acetamido-3-((2-((4Z,7Z,10Z,13Z, 16Z, 19Z)-docosa-4,7,10,13,16,19-hexaenamido)ethyl)amino)-3-oxopropyl)disulfanyl)propanoate **(I-71)** 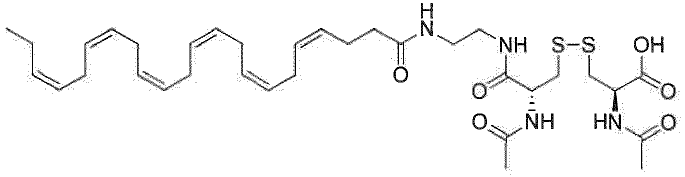 (R)-2-acetamido-3-(((R)-2-acetamido-3-((2-((4Z,7Z,10Z,13Z,16Z,19Z)-docosa-4,7,10,13,16,19-hexaenamido)ethyl)amino)-3-oxopropyl)disulfanyl)propanoic acid **(I-72);** 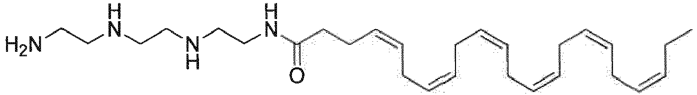 (4Z,7Z,10Z,13Z,16Z,19Z)-N-(2-((2-((2-aminoethyl)amino)ethyl)amino)ethyl)docosa-4,7,10,13,16,19-hexaenamide **(I-73);** 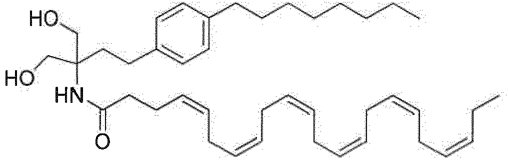 (4Z,7Z,10Z,13Z,16Z,19Z)-N-(1-hydroxy-2-(hydroxymethyl)-4-(4-octylphenyl)butan-2-yl)docosa-4,7,10,13,16,19-hexaenamide **(I-74);** 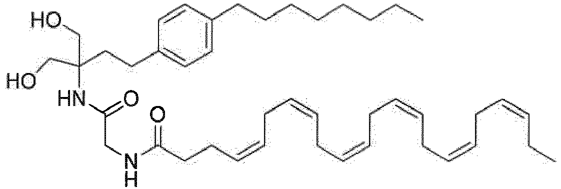 (4Z,7Z,10Z,13Z,16Z,19Z)-N-(2-((1-hydroxy-2-(hydroxymethyl)-4-(4-octylphenyl)butan-2-yl)amino)-2-oxoethyl)docosa-4,7,10,13,16,19-hexaenamide **(I-75);** 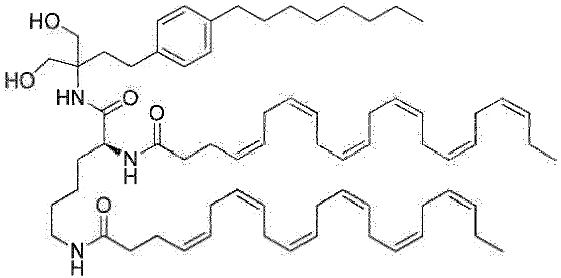 (4Z,4'Z,7Z,7'Z,10Z,10'Z,13Z,13'Z,16Z,16'Z,19Z,19'Z)-N,N'-((S)-6-((1-hydroxy-2-(hydroxymethyl)-4-(4-octylphenyl)butan-2-yl)amino)-6-oxohexane-1,5-diyl)bis(docosa-4,7,10,13,16,19-hexaenamide) **(I-76);** 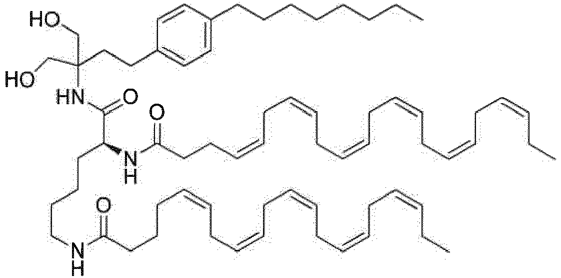 (4Z,7Z,10Z,13Z,16Z,19Z)-N-((S)-1-((1-hydroxy-2-(hydroxymethyl)-4-(4-octylphenyl)utan-2-yl)amino)-6-((5Z,8Z,11Z,14Z,17Z)-icosa-5,8,11,14,17-pentaenamido)-1-oxohexan-2-yl)docosa-4,7,10,13,16,19-hexaenamide **(I-77);** 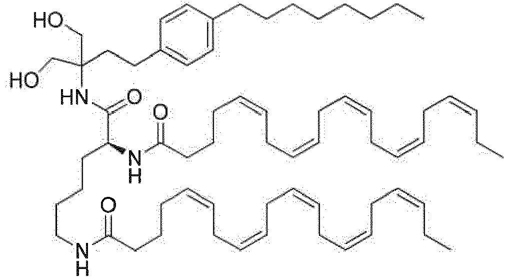 (5Z,5'Z,8Z,8'Z,11Z,11'Z,14Z,14'Z,17Z,17'Z)-N,N'-((S)-6-((1-hydroxy-2-(hydroxymethyl)-4-(4-octylphenyl)butan-2-yl)amino)-6-oxohexane-1,5-diyl)bis(icosa-5,8,11,14,17-pentaenamide) (1-78); 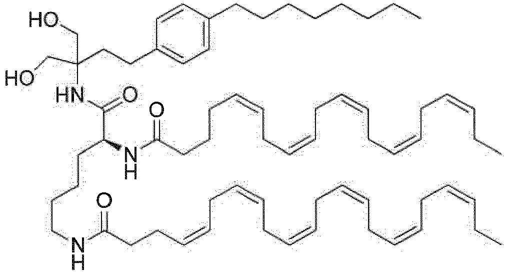 (4Z,7Z,10Z,13Z,16Z,19Z)-N-((S)-6-((1-hydroxy-2-(hydroxymethyl)-4-(4-octylphenyl)butan-2-yl)amino)-5-((5Z,8Z,11Z,14Z,17Z)-icosa-5,8,11,14,17-pentaenamido)-6-oxohexyl)docosa-4,7,10,13,16,19-hexaenamide (1-79); 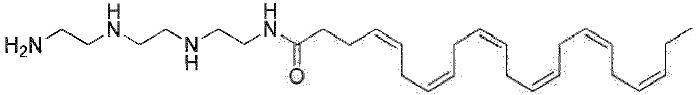 (4Z,7Z,10Z,13Z,16Z,19Z)-N-(2-((2-((2-aminoethyl)amino)ethyl)amino)ethyl)docosa-4,7,10,13,16,19-hexaenamide (1-80); 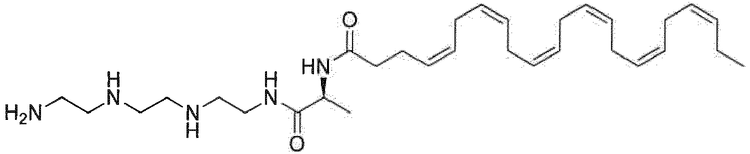 (4Z,7Z,10Z,13Z,16Z,19Z)-N-((S)-1-((2-((2-((2-aminoethyl)amino)ethyl)amino)ethyl)amino)-1-oxopropan-2-yl)docosa-4,7,10,13,16,19-hexaenamide (**I-81**). 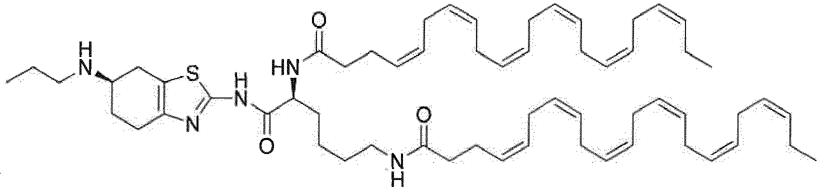 (4Z,4'Z,7Z,7'Z,10Z,10'Z,13Z,13Z,16Z,16'Z,19Z,19'Z)-N,N'-((S)-6-oxo-6-(((R)-6-(propylamino)-4,5,6,7-tetrahydrobenzo[d]thiazol-2-yl)amino)hexane-1,5-diyl)bis(docosa-4,7,10,13,16,19-hexaenamide) **(I-82)**

### Methods for using fatty acid amides

Also provided in the invention is a compound of the invention for use in a method for inhibiting, preventing, or treating inflammation or an inflammatory disease in a subject, the inflammatory disease being selected from an autoimmune disease, an inflammatory respiratory disease, or a neurodegenerative disease. Examples of such diseases include, but are not limited to: rheumatoid arthritis, asthma, adult respiratory distress syndrome, chronic obstructive airway disease, and cystic fibrosis; psoriasis, multiple sclerosis, Alzheimer's disease, Parkinson's disease, Huntington's disease, amyotrophic lateral sclerosis and viral or autoimmune encephalitis.

In some embodiments, the subject is administered an effective amount of a fatty acid amide.

The fatty acid amides can each be administered in amounts that are sufficient to treat or prevent a metabolic disease or prevent the development thereof in subjects.

Administration of the fatty acid amides can be accomplished via any mode of administration for therapeutic agents. These modes include systemic or local administration such as oral, nasal, parenteral, transdermal, subcutaneous, vaginal, buccal, rectal or topical administration modes.

Depending on the intended mode of administration, the compositions can be in solid, semi-solid or liquid dosage form, such as, for example, injectables, tablets, suppositories, pills, time-release capsules, elixirs, tinctures, emulsions, syrups, powders, liquids, suspensions, or the like, sometimes in unit dosages and consistent with conventional pharmaceutical practices. Likewise, they can also be administered in intravenous (both bolus and infusion), intraperitoneal, subcutaneous or intramuscular form, all using forms well known to those skilled in the pharmaceutical arts.

Illustrative pharmaceutical compositions are tablets and gelatin capsules comprising a fatty acid amide and a pharmaceutically acceptable carrier, such as: a) a diluent, e.g., purified water, triglyceride oils, such as hydrogenated or partially hydrogenated vegetable oil, or mixtures thereof, corn oil, olive oil, sunflower oil, safflower oil, fish oils, such as EPA or DHA, or their esters or triglycerides or mixtures thereof, omega-3 fatty acids or derivatives thereof, lactose, dextrose, sucrose, mannitol, sorbitol, cellulose, sodium, saccharin, glucose and/or glycine; b) a lubricant, e.g., silica, talcum, stearic acid, its magnesium or calcium salt, sodium oleate, sodium stearate, magnesium stearate, sodium benzoate, sodium acetate, sodium chloride and/or polyethylene glycol; for tablets also; c) a binder, e.g., magnesium aluminum silicate, starch paste, gelatin, tragacanth, methylcellulose, sodium carboxymethylcellulose, magnesium carbonate, natural sugars such as glucose or beta-lactose, corn sweeteners, natural and synthetic gums such as acacia, tragacanth or sodium alginate, waxes and/or polyvinylpyrrolidone, if desired; d) a disintegrant, e.g., starches, agar, methyl cellulose, bentonite, xanthan gum, alginic acid or its sodium salt, or effervescent mixtures; e) absorbent, colorant, flavorant and sweetener; f) an emulsifier or dispersing agent, such as Tween 80, Labrasol, HPMC, DOSS, caproyl 909, labrafac, labrafil, peceol, transcutol, capmul MCM, capmul PG-12, captex 355, gelucire, vitamin E TGPS or other acceptable emulsifier; and/or g) an agent that enhances absorption of the compound such as cyclodextrin, hydroxypropyl-cyclodextrin, PEG400, PEG200.

Liquid, particularly injectable, compositions can, for example, be prepared by dissolution, dispersion For example, the fatty acid amide is dissolved in or mixed with a pharmaceutically acceptable solvent such as, for example, water, saline, aqueous dextrose, glycerol, ethanol, and the like, to thereby form an injectable isotonic solution or suspension. Proteins such as albumin, chylomicron particles, or serum proteins can be used to solubilize the fatty acid amides.

The fatty acid amides can be also formulated as a suppository that can be prepared from fatty emulsions or suspensions; using polyalkylene glycols such as propylene glycol, as the carrier.

The fatty acid amides can also be administered in the form of liposome delivery systems, such as small unilamellar vesicles, large unilamellar vesicles and multilamellar vesicles. Liposomes can be formed from a variety of phospholipids, containing cholesterol, stearylamine or phosphatidylcholines. In some embodiments, a film of lipid components is hydrated with an aqueous solution of drug to a form lipid layer encapsulating the drug, as described in United States Patent No. 5,262,564.

Fatty acid amides can also be delivered by the use of monoclonal antibodies as individual carriers to which the fatty acid amides are coupled. The fatty acid amides can also be coupled with soluble polymers as targetable drug carriers. Such polymers can include polyvinylpyrrolidone, pyran copolymer, polyhydroxypropylmethacrylamide-phenol, polyhydroxyethylaspanamidephenol, or polyethyleneoxidepolylysine substituted with palmitoyl residues. Furthermore, the fatty acid amide conjugates can be coupled to a class of biodegradable polymers useful in achieving controlled release of a drug, for example, polylactic acid, polyepsilon caprolactone, polyhydroxy butyric acid, polyorthoesters, polyacetals, polydihydropyrans, polycyanoacrylates and cross-linked or amphipathic block copolymers of hydrogels. In one embodiment, fatty acid amides are not covalently bound to a polymer, e.g., a polycarboxylic acid polymer, or a polyacrylate.

Parenteral injectable administration is generally used for subcutaneous, intramuscular or intravenous injections and infusions. Injectables can be prepared in conventional forms, either as liquid solutions or suspensions or solid forms suitable for dissolving in liquid prior to injection.

Compositions can be prepared according to conventional mixing, granulating or coating methods, respectively, and the present pharmaceutical compositions can contain from about 0.1 % to about 80 %, from about 5 % to about 60 %, or from about 1 % to about 20 % of the fatty acid amide conjugate by weight or volume.

The dosage regimen utilizing the fatty acid amide is selected in accordance with a variety of factors including type, species, age, weight, sex and medical condition of the patient; the severity of the condition to be treated; the route of administration; the renal or hepatic function of the patient; and the particular fatty acid amide conjugate employed. A physician or veterinarian of ordinary skill in the art can readily determine and prescribe the effective amount of the drug required to prevent, counter or arrest the progress of the condition.

Effective dosage amounts of the present invention, when used for the indicated effects, range from about 20 mg to about 5,000 mg of the fatty acid amides per day. Compositions for *in vivo* or *in vitro* use can contain about 20, 50, 75, 100, 150, 250, 500, 750, 1,000, 1,250, 2,500, 3,500, or 5,000 mg of the fatty acid amide. In one embodiment, the compositions are in the form of a tablet that can be scored. Effective plasma levels of the fatty acid amide can range from about 5 ng/mL to 5000 ng/mL per day. Appropriate dosages of the fatty acid amides can be determined as set forth in Goodman, L. S.; Gilman, A. The Pharmacological Basis of Therapeutics, 5th ed.; MacMillan: New York, 1975, pp. 201-226.

Fatty acid amides can be administered in a single daily dose, or the total daily dosage can be administered in divided doses of two, three or four times daily. Furthermore, fatty acid amides can be administered in intranasal form via topical use of suitable intranasal vehicles, or via transdermal routes, using those forms of transdermal skin patches well known to those of ordinary skill in that art. To be administered in the form of a transdermal delivery system, the dosage administration can be continuous rather than intermittent throughout the dosage regimen. Other illustrative topical preparations include creams, ointments, lotions, aerosol sprays and gels, wherein the concentration of the fatty acid amide conjugate ranges from about 0.1 % to about 15 %, w/w or w/v.

### Combination Therapies

Fatty acid amides may also be administered with other therapeutic agents such as cholesterol-lowering agents, fibrates and hypolipidemic agents, DPP-IV inhibitors as anti-diabetic agents, anti-diabetic agents, antiepileptic agents, antiglaucoma agents, antihypertensive agents, anti-inflammatory agents, TNF-α inhibitors, anti-depressant agents, anti-cancer agents, immunosuppressant agents, agents to treat osteoporosis, and agents to treat multiple sclerosis. In some embodiments, the fatty acid amide can be co-administered with the other therapeutic agent. In some embodiments, the fatty acid amide can be administered before the other therapeutic agent. In some embodiments, the fatty acid amide can be administered after the other therapeutic agent.

In some embodiments, the other therapeutic agent is a cholesterol-lowering agent. Examples of cholesterol-lowering agents are atorvastatin, cerivastatin, fluvastatin, lovastatin, pitavastatin, pravastatin, rosuvastatin, simvastatin, ezetimibe, and the combination of ezetimibe/simvastatin (Vytorin®).

In some embodiments, the other therapeutic agent is a fibrate or hypolipidemic agent. Examples of fibrates or hypolipidemic agents are acifran, acipimox, beclobrate, bezafibrate, binifibrate, ciprofibrate, clofibrate, colesevelam, gemfibrozil, fenofibrate, melinamide, niacin, and ronafibrate.

In some embodiments, the other therapeutic agent is a DPP-IV inhibitor as anti-diabetic agent. Examples of DPP-IV inhibitors as anti-diabetic agents are sitagliptin, saxagliptin, vildagliptin, linagliptin, dutogliptin, gemigliptin and alogliptin.

In some embodiments, the other therapeutic agent is an Anti-diabetic agent. Examples of anti-diabetic agents are acarbose, epalrestat, exenatide, glimepiride, liraglutide, metformin, miglitol, mitiglinide, nateglinide, pioglitazone, pramlintide, repaglinide, rosiglitazone, tolrestat, troglitazone, and voglibose.

In some embodiments, the other therapeutic agent is an antiepileptic agent. Examples of antiepileptic agents include Gabapentin, pregabalin.

In some embodiments, the other therapeutic agent is an Antiglaucoma agents. Examples of antiglaucoma agents include apraclonidine, befunolol, bimatroprost, brimonidine, brinzolamide, dapiprazole, dorzolamide, latanoprost, levobunolol, tafluprost, travoprost, and unoprostone isopropyl ester.

In some embodiments, the other therapeutic agent is an antihypertensive agents. Examples of antihypertensive agents include alacepril, alfuzosin, aliskiren, amlodipine besylate, amosulalol, aranidipine, arotinolol HCl, azelnidipine, bamidipine hydrochloride, benazepril hydrochloride, benidipine hydrochloride, betaxolol HCl, bevantolol HCl, bisoprolol fumarate, bopindolol, bosentan, budralazine, bunazosin HCl, candesartan cilexetil, captopril, carvedilol, celiprolol HCl, cicletanine, cilazapril, cinildipine, clevidipine, delapril, dilevalol, doxazosin mesylate, efonidipine, enalapril maleate, enalaprilat, eplerenone, eprosartan, felodipine, fenoldopam mesylate, fosinopril sodium, guanadrel sulfate, imidapril HCl, irbesartan, isradipine, ketanserin, lacidipine, lercanidipine, lisinopril, losartan, manidipine hydrochloride, mebefradil hydrochloride, moxonidine, nebivolol, nilvadipine, nipradilol, nisoldipine, olmesartan medoxomil, perindopril, pinacidil, quinapril, ramipril, rilmedidine, spirapril HCl, telmisartan, temocarpil, terazosin HCl, tertatolol HCl, tiamenidine HCl, tilisolol hydrochloride, trandolapril, treprostinil sodium, trimazosin HCl, valsartan, and zofenopril calcium.

In some embodiments, the other therapeutic agent is an anti-inflammatory agent. Examples of anti-inflammatory agents include celecoxib, rofecoxib, ibuprofen, naproxen, indomethacin, salicylic acid, salsalate, 5-aminosalicylic acid, dimethylfumarate, monomethyl fumarate, methotrexate, predisone, prednisolone, abatecept, aceclofenac, AF-2259, alefacept, amfenac sodium, ampiroxicam, amtolmetin guacil, arformoterol, bambuterol, bardoxolone methyl, butibufen, cankinumab, ciclesonide, deflazacort, doxofylline, dexibuprofen, droxicam, etodolac, flunoxaprofen, fluticasone propionate, fomoterol fumarate, golimumab, indacaterol, interferon-gamma, isofezolac, isoxicam, lobenzarit sodium, lomoxicam, loxoprofen sodium, lumiracoxib, mabuterol HCl, nabumetone, nepafenac, nimesulide, oxaprozin, oxitropium bromide, piroxicam cinnamate, rimexolone, sivelestat, tenoxicam, zaltoprofen, fisalamine, and osalazine.

In some embodiments, the other therapeutic agent is a TNF-α inhibitor. Examples of TNF-α inhibitors include infliximab, adalimumab, certolizumab, golimumab, and etanercept.

In some embodiments, the other therapeutic agent is an anti-depressant agents. Examples of anti-depressant agents include bupropion HCl, citalopram, desvenlafaxine, fluoxetine HCl, fluvoxamine maleate, metapramine, milnacipran, mirtazapine, moclobemide, nefazodone, paroxetine, pivagabine, reboxetine, setiptiline, sertraline HCl, tianeptine sodium, toloxatone and venlafaxine.

In some embodiments, the other therapeutic agent is an anti-cancer agent. Examples of anti-cancer agents include abarelix, alemtuzumab, alitretinoin,amrubicin HCl, amsacrine, anastrozole, arglabin, azacitidine, belotecan, bevacizumab, bexarotene, bicalutamide, bisantrene HCl, bortezomib, camostat mesylate, capecitabine, catumaxomab, cetuximab, cladribine, clofarabine, cytarabine ocfosfate, dasatinib, degarelix acetate, denileukin diftitox, doxetaxel, doxifluridine, enocitabine, epirubicin HCl, erlotinib, exemestane, fludarabine phosphate, flutamide, formestane, fotemustine, fulvestrant, geftimib, gemcitabine HCl, gemtuzumab ozogamicin, ibritumomab tiuxetan, idarubicin HCl, imatibib mesylate, interferon gamma-1a, interleukin-2, imotecan, INCB18424, ixabepilone, lapatinib, lenalidomide, letrazole, lonidamine, mitoxantrone HCl, nelarabine, nedaplatin, nilutamide, nimotuzumab, OCT-43, ofatumumab, oxaliplatin, paclitaxal, panitumumab, pazopanib, pegaspargase, pemetrexed, pentostatin, pirarubicin, pralatrexate, raltitrexed, ranimustine, ridaforolimus, SKI-2053R, sobuzoxane, sorafenib, sunitinib, talaporfin sodium, tamibarotene, tasonermin, temoporphin, temozolomide, temsirolimus, topotecan HCl, toremifene, tosimomab, trabectedin, valrubicin,vinorelbine, vorinostat and zinostatin stimalamer.

In some embodiments, the other therapeutic agent is an immunosuppressant agent. Examples of immunosuppressant agents include cyclosporine, everolimus, gusperimus, mizoribine, muromonab-CD3, mycophenolate sodium, mycophenolate mofeti, pimecrolimus, tacrolimus.

In some embodiments, the other therapeutic agent is an agents to treat osteoporosis. Examples of agents to treat osteoporosis include alendronate sodium, ibandronic acid, incadronic acid, raloxifene HCl, risdronate sodium, strontium ranelate.

In some embodiments, the other therapeutic agent is an agent to treat multiple sclerosis. Examples of agents to treat multiple sclerosis include dimethyl fumarate, mono methyl fumarate, fingolimod, teriflunomide, laquinimod, cladribine, interferon beta-1a, betaseron, glatimer acetate, natalizumab.

### METHODS OF MAKING

Examples of synthetic pathways useful for making Fatty Acid Conjugates of Formula **I** are set forth in the Examples below and generalized in **Schemes 1-6.** 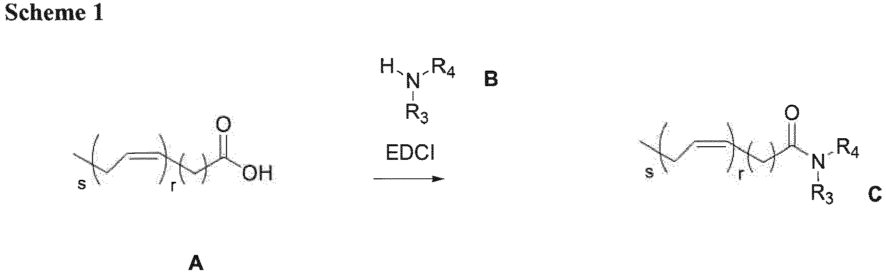 wherein r, s, R₃ and R₄ are as defined above.

A fatty acid of the general formula **A** can be coupled directly with an amine of the general formula **B** using any standard amide coupling reagent such as EDCI, HATU or DCC, in an organic solvent such as CH₂Cl₂ or acetonitrile. A large variety of amines of the formula **B** is commercially available. 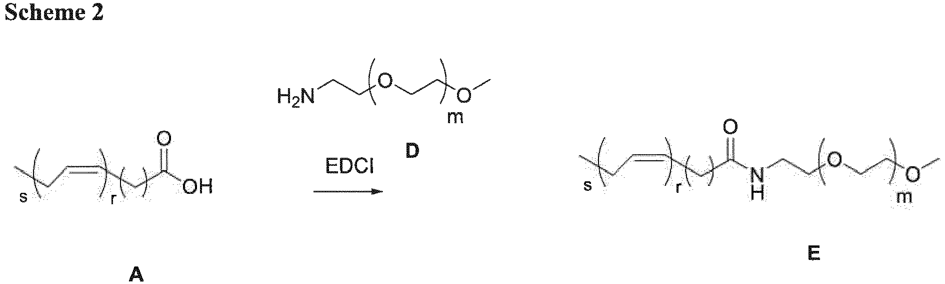 wherein r, s and m are as defined above.

A fatty acid of the general formula **A** can be coupled directly with an amine of the general formula **D** using any standard amide coupling reagent such as EDCI, HATU or DCC, in an organic solvent such as CH₂Cl₂ or acetonitrile. A number of these polyether amines (wherein m is 1, 2, 3, 4, 5 and 6) are commercially available. To those familiar in the art, instead of the amine D, primary amines such as the ones shown below (all commercially available) can be used for the same amide coupling reaction: 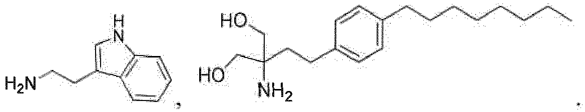 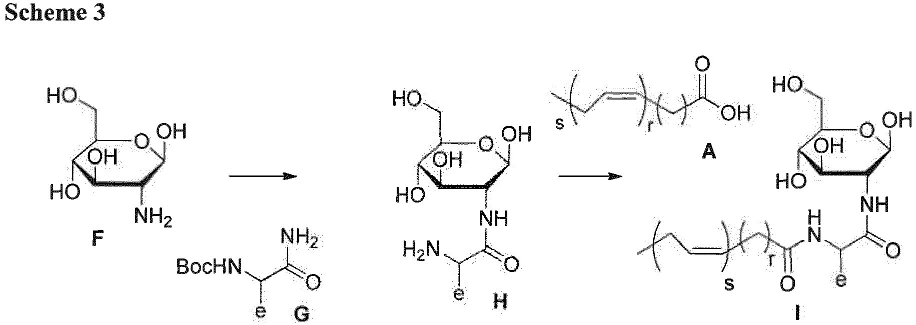 wherein e, r, and s are as defined above.

The commercially available amine **F** can be coupled with a BOC-protected amino acid of the formula **G** using EDC or HATU to afford an intermediate amide. The BOC protecting group can be removed by treatment with acid such TFA or HCl to afford compounds of the general formula **H.** This amine can be coupled with a fatty acid of the general formula **A** to obtain compounds of the general formula **I.** To those familiar in the art, any other amines such as the ones shown in Scheme 3 can be used for the same sequence of reactions. 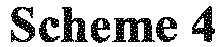 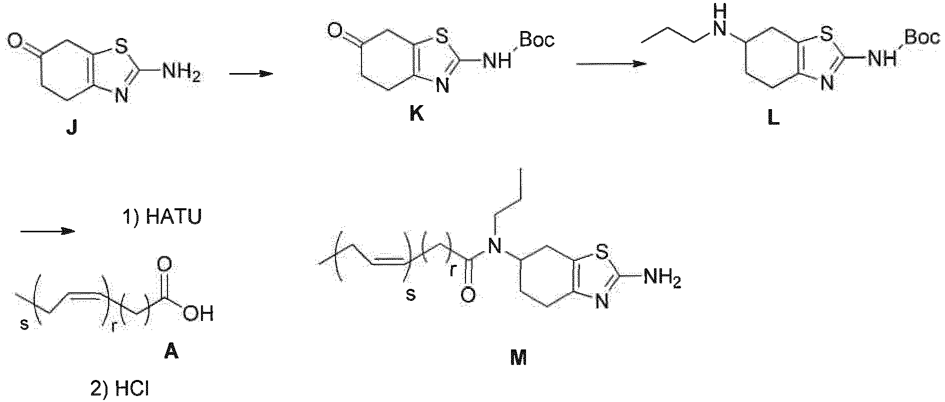 wherein r and s are as defined above.

The commercially available amine **J** is treated with a reagent such as BOC-anhydride to obtain the BOC-protected derivative **K.** A reductive amination using propylamine in the presence of either sodium triacetoxyborohydride or sodium cyanoborohydride affords compound **L,** a differentially protected diamine intermediate. Intermediate **L** can be coupled with a fatty acid of the formula **A** in the presence of HATU and a tertiary amine such as DIEA or Et₃N, followed by treatment with HCl to afford compound **M.** 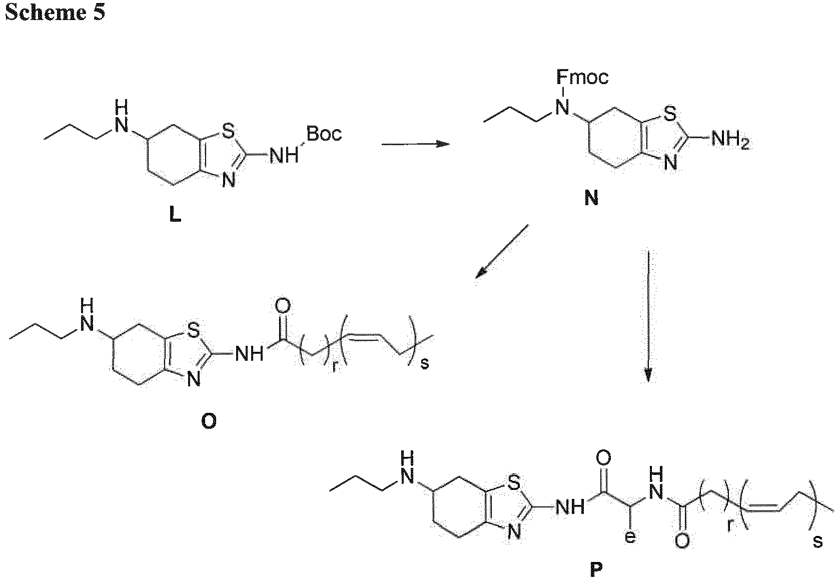 wherein e, r and s are as defined above.

To those familiar in the art, this scheme illustrates the use of differential protecting group to enable amide coupling reaction at one of the two amino sites. The BOC-protected amine **L** can be treated with a reagent such as FMOC-Cl according to the procedures outlined in Greene's Protective Groups in Organic Synthesis, Wiley, 3rd Edition. The resulting Fmoc-protected compound can be treated with acids such as TFA or HCl to afford compound **N.** Compound **N** can be coupled with a fatty acid of the formula **A**, followed by treatment with a base such as morpholine or diethylamine to obtain compounds of the general formula **O.** To those familiar in the art, compound **N** can also be subjected to the same reaction sequence outlined in Scheme 3 using the BOC-protected amino acid of the formula **G** to eventually obtain compounds of the general formula **P.** 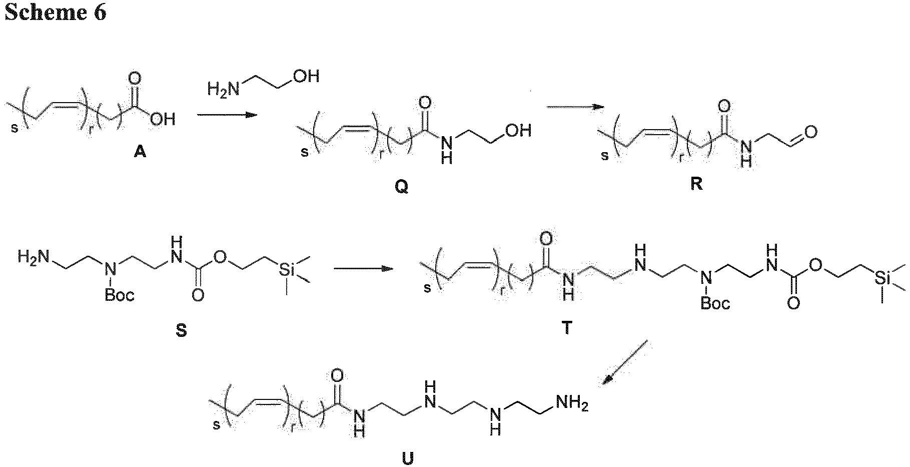 whewherein r and s are as defined above.

To those familiar in the art, Scheme 6 illustrates the use of protecting groups to differentiate a polyamine. The fatty acid of the formula **A** is coupled with ethanolamine to obtain compounds of the general formula **Q.** The alcohol group can be oxidized to the corresponding aldehyde using reagents such as pyridinium dichromate or Dess-Martin reagent to obtain compounds of the general formula **R.** The acylated amine of the formula **S** can be prepared using the procedures outlined in Andruszkiewicz et al. Synthetic Commun. 2008, 38, 905-913. Compound **R** and **S** can be reacted under reductive amination conditions using either sodium borohydride or sodium cyanoborohydride to obtain compounds of the general formula **T.** Treatment of **T** with a fluoride reagent will selectively remove the silyl protecting group and treatment with an acid such as TFA or HCl will remove the remaining BOC protecting group to afford compounds of the general formula **U.**

### EXAMPLES

The disclosure is further illustrated by the following examples.

The following compound examples serve to illustrate further embodiments of the fatty acid amides. Examples not falling within the scope of the claims are for illustrative purposes only.

### Example 1

### Effect of the compounds of the invention in cystinotic fibroblasts

Nephropathic cystinosis is an orphan genetic lysosomal storage disorder characterized by a massive accumulation of crystalline cystine within cells. Affected individuals develop a proximal renal tubulopathy and progress, if untreated, to end-stage renal failure. Omran et al (Bioorg. Med. Chem. 2011, p, 3492) described a convenient in vitro system to quickly evaluate compounds that could deplete levels of cystine in cultured fibroblasts. Cystinotic fibroblasts (cell line GM00008, Coriell Cell Repository) were seeded in a 6-well plate and grown in DMEM/15% FBS until confluent. The compound of the invention was formulated in 100% ethanol at a concentration of 50 mM; prior to addition to cells, drugs were further diluted in 100% FBS and sonicated for one hour. Cells were incubated with drug for 24 hours, then harvested via trypsinization. Cells were subsequently washed in PBS and suspended in a 3:1 solution of 5.2 mM N-ethylmaleimide (Sigma) to 12% sulfosalicylic acid (Sigma) in order to derivatize free cysteine and precipitate protein respectively. To lyse cells, samples were then frozen at -80 degrees Celsius, thawed, and sonicated twice for 1 minute. Lysates were cleared of precipitated protein via centrifugation and samples were subsequently processed for LC-MS/MS analysis.

Protein quantification was accomplished by dissolving precipitated protein in 0.25 M NaOH and 1 M Tris (pH 7.5), and spectrophotometric measurement using the Bradford method.

Compound **I-9** was evaluated in this cystinotic fibroblast assay. The level of cystine in this cystinotic cell line was quantitated by LC-MS/MS. A depletion of cystine was observed when cystinotic fibroblasts were treated with 50 µM of compound **I-9.** The level of cystine was expressed as the percentage of control (POC). Using this method, compound **I-9** showed a 68% reduction in the cystine level at 50 µM, whereas the control compound cysteamine showed a 45% reduction in the cystine level at 50 µM. The cystine level was determined as follows: To the 50 µL of supernatant, acetonitrile (400µL) containing 200ng/ml Internal standard (deuterated cystine) was added and mixture was vortexed for 1 min followed by centrifugation for 10 minutes. Supernatant was transferred to a clean LC vials and 10 µl of the sample was injected on LC-MS/MS for the measurement of Cystine.

### LC-MS analysis

The concentration of Cystine was measured by LC-MS/MS. 10µl of the sample was injected into the column (Luna, HILIC, 150X4.60mm, 3µ Phenomenex). Cystine and deuterated cystine was eluted from the column by a stepwise gradient of 10% mobile phase B at 0 min, 10% B at 2.5 min, 50% B at 2.7 min and 10% B at 3 min. Mobile phase A contained Acetonitrile/Water/Ammonium acetate (20mM) with 0.1% Formic acid and mobile phase B contained Acetonitrile/Ammonium acetate (20mM) with 0.1% formic acid. The column elute was directly injected into a Agilent triple quad, which was maintained in electrospray positive mode. The retention times for Cystine and the internal standard (deuterated cystine) was 2.25 min. Cystine was monitored via the transition m/z 241.3-152 with a fragmentor of 75 and collision energy of 7Ev and duterated cystine was monitored via the transition m/z 247.3-124.1 with a fragmentor 126 and collision energy of 9eV. The column was maintained at 25°C.

### Example 2

### Effect of the compounds of the invention in a genetic mouse model lacking cystinosin, the protein defective in nephropathic cystinosis.

Nephropathic cystinosis is an orphan genetic lysosomal storage disorder characterized by a massive accumulation of crystalline cystine within cells. The accumulation of cysteine is due to a defective lysosomal cystine transporter called cystinosin. A genetic mouse model lacking cystinosin has recently been described (Cherqui et al Molecular and Cellular Biology 2002, 22, p. 7622-7632). Mice lacking cystinosin have been shown to have a substantial increase in cystine level in numerous tissues including liver, kidney, spleen, muscle, brain and heart. Treatment of mice lacking cystinosin with cysteamine at 200 or 400 mg/kg orally for 7, 30 or 60 days has been shown to reduce cystine levels of all tissues tested, with the greatest effect observed in the liver and kidney, after 60 days. Compounds described herein can be evaluated in a similar fashion for their ability to reduce cystine levels in certain tissues using this genetic mouse model lacking cystinosin.

### Example 3

### Effect of the compounds of the invention in the transgenic YAC128 mouse model for Huntington disease

Transgenic Huntington disease mice expressing highly expanded human huntingin (YAC128) and their wild type littermates can be used to assess the effect of bis-fatty acid conjugates containing cystamine on transglutamine levels. Three-month old YAC mice are treated with the desired conjugate for a period of two weeks before being sacrificed. Mice are asphyxiated with carbon dioxide and the brains are immediately removed, separated into forebrain and hindbrain, frozen in isopentane on dry ice and stored at -80 °C. Tissues are then processed to measure transglutaminase activity as well as to assess striatal neuropathology according to the detailed procedure outlined in Van Raamsdock, J. M. et al, J. Neurochemistry 2005, 95, p. 210-220. Transgenic animals that are 7 months of age can also be used to assess for motor functions (such as time on rotarod) when administered with compounds of this invention over a period of 4 months.

### Example 4

### Effect of fatty acid amides on ApoB secretion in HepG2 cells

HepG2 cells (ATCC) are seeded at 25,000 cells per well in collagen-coated 96-well plates in growth media (DMEM with 10% fetal bovine serum). The following day, fatty acid amides are complexed to lipoprotein-deficient fetal bovine serum at the appropriate concentration. Growth media is then removed from and the HepG2 cells are washed once with PBS. The lipoprotein-deficient FBS with the complexed fatty acid amide conjugates is added to DMEM for a final 10% concentration. Each concentration of fatty acid amide is tested in triplicate. Cells are incubated for 16 hours with the fatty acid amide. Alamar Blue® (Invitrogen) is then added to the media to determine cell viability per the manufacturer's instructions. Two hours after Alamar Blue® addition, the media is removed and placed in a black 96-well plate. The plate is then read at 550nm/590nm to determine cell viability. The media is then used to determine ApoB concentrations using ELISA kits (Mabtech AB). Percent inhibition of ApoB secretion is determined by normalizing data to vehicle treated wells. For a given compound, an IC₅₀ (concentration at which 50% of ApoB secretion is inhibited) can also be determined by using a 4 parameter-fit inhibition curve model (Graph Pad Prism®).

### Example 5

### Effect of fatty acid amides on SREBP-1c target genes

HepG2 cells (ATCC) are seeded at 20,000 cells per well in 96 well plates. After adhering overnight, growth media (10% FBS in DMEM) is removed and cells are serum starved for 24 hours in DMEM containing 1% fatty acid free bovine serum albumin (BSA, Sigma). Cells are then treated with the fatty acid amides at a final concentration of 50 µM in 1% BSA or 0.1 oleate complexed to fatty acid free BSA in a 5:1 molar ratio. Cells are incubated for 6 hours and then washed with PBS. RNA was reverse-transcribed using the cells II cDNA reagents according to standard protocols (outlined in Applied Biosystem StepOne Real-time PCR protocols). Real time PCR of transcripts can be performed with Tagman assays for the three specific genes FASN (fatty acid synthase), SCD (steroyl CoA desaturase) and ApoA1(apolipoprotein A1). In all three cases, 18S-VIC® is used as a normalization control.

### Example 6

### Effect of fatty acid amides in the Zucker fa/fa rat model

Male Zucker rats (*HsdHlr:ZUCKER-Lepr^fa)* between 8-10 weeks of age are purchased from Harlan. Zucker rats are maintained on Teklad Global Rodent Diet (2018S) during the acclimation period and for the duration of study. The Zucker rats are weighed and randomly assigned to treatment arms based on body weight and plasma TG levels (n is 8). Inclusion criteria for the study include body weight >300 grams and fed TG levels in plasma >800 mg/dL. Rats are randomized into treatment arms based on pre-dose (day -1) body weights and plasma levels (fed) of triglycerides. Dosing is initiated on day 1 and continue through day 5. Dosing is daily (qd) by oral gavage (po) for all treatment arms. Body weights are measured for all rats on days 1 through 5. On day 4, a blood sample (fed) are collected from each rat, processed for plasma and stored at -80°C. At 8pm on day 4 food are removed from all rats to initiate fasting state. On day 5 rats are dosed at 8am according to treatment arm. Two hours later (10am) two blood draws from each rat are collected and processed for plasma. Triglyceride levels are then analyzed by standard protocols using commercially available kits.

### Example 7

### Effect of fatty acid amides in the Golden Syrian Hamster model of dyslipidemia.

Golden Syrian Hamster (Strain: HsdHan™:AURA, from Harlan Laboratories), 5-6 weeks of age, with a body weight of approximately 80 g, are used for the study. The Hamsters are maintained on high fat diet D12492 (Research Diets, New Brunswick NJ) during the acclimation period and throughout the study. Animals will then receive drinking water supplemented with 10% fructose (Sigma, supplied by Catabasis) starting on day -8 and continuing throughout the study. The hamsters will be randomized into treatment arms based on pre-dose (day -1) body weights and plasma levels (fed) of triglycerides (TG). Dosing will be initiated on day 1 and continue through day 28. Dosing will be daily (qd) by oral gavage (po) for all treatment arms. On day 27, hamsters will be fasted at the beginning of the dark cycle. Hamsters will be dosed at 8am on day 28 according to the treatment arm. Two hours later (10am) a blood sample will be collected from each hamster, processed to plasma and stored at -80°C. Triglyceride and HDL cholesterol levels will be determined using standard protocols and the commercially available kits from Abcam, Cayman or Sigma-Aldrich.

### Example 8

### Effects of compounds of the invention on NF-κB Levels in RAW 264.7 Macrophages

RAW 264.7 cells stably expressing a 3x NF-κB response elemement-drive luciferase reporter were seeded into 96 well plates in sera-free medium (Optimem) 18 hours prior to compound application. Compounds of the invention were prepared by first making 100 mM stock solutions in EtOH. Stock solutions were then diluted 1:100 in low LPS FBS (Gemini BenchMark 100-106), mixed vigorously and allowed to incubate at room temperature for 30 minutes. 1:2 serial dilutions were then made in FBS supplemented with 1% EtOH, mixed vigorously, and again allowed to incubate at room temperature for 30 minutes before adding to RAW 264.7 reporter cells (final concentrations: 10% FBS, 100uM highest compound dilution, 0.1% EtOH) for a 2 hour pretreatment prior to stimulation with LPS. Cells were then stimulated with 200 ng/ml LPS or vehicle control for 3 hours in the presence of the compounds of the invention. A set of six vehicles was left unstimulated with LPS in order to measure the assay floor. AlamarBlue viability dye (Invitrogen) was added to cells simultaneously with the delivery of LPS (final AlamarBlue concentration of 10%). After the 3 h incubation period with LPS, cell viability was measured by reading fluorescence (excitation 550 nm, emission 595 nm) with a Perkin Elmer Victor V plate reader. Then cell media was aspirated from each well. Luciferase signal was then developed by addition of the Britelite Plus reagent (Perkin Elmer). Luciferase activity was measured with the Perkin Elmer Victor V plate reader. NF-κB activity was expressed as a percent of the vehicle control wells (stimulated with LPS). Compounds were tested at 6 dose point titrations in triplicate to determine IC₅₀ values. Table 1 summarizes the IC50 values for a number of fatty acid amides in this NF-κB luciferase reporter assay. A (-) indicates that the compound showed no inhibitory activity up to 200 µM. A (+) indicates that the compound showed inhibitory activity of less than 200 µM. A (+ +) indicates that the compound showed inhibitory activity of less than 50 µM.

**Table 1. Summary of IC50 values for fatty acid amides in the NF-κB luciferase reporter assay.**

| **Compound** | **NF-kB inhibitory activity IC₅₀ µM** |
|---|---|
| DHA | - |
| EPA | - |
| **I-21** | + |
| **I-47** | - |
| **I-48** | ++ |
| **I-49** | ++ |
| **I-52** | ++ |
| **I-57** | ++ |

### Example 9

### Effect of fatty acid amides on IL-1β

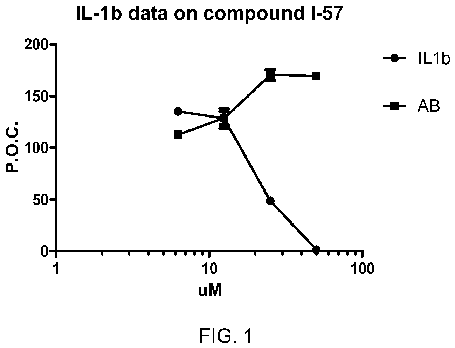

RAW264.7 macrophages were seeded at a density of 100,000 cells/well in a 96-well plate in DMEM supplemented with 10% FBS and Penn/strep. 16 hours later, medium was aspirated and replaced with 90µL/well of serum-free DMEM. Fatty acid amides were brought up in 100% EtOH to a concentration of 100mM and then diluted 1:100 in 100% FBS for a stock solution consisting of ImM compound and 1% EtOH. These stock solutions were then diluted 1:10 in FBS supplemented with 1% EtOH to generate a 100 µM of the fatty acid amide. 10µL was then added to the RAW246.7 cells to generate final concentrations 10µM of the fatty acid amide or 10µM each DHA and MMF, along with vehicle only control. The compounds were allowed to pre-incubate for 2 hours before stimulation of 100ng/ml LPS (10µL of 1µg/ml LPS was added to each well). Following 3 hours of LPS stimulation, cells were washed once in 1x PBS, aspirated dry, and flash frozen in liquid nitrogen. RNA was then isolated and converted to cDNA using the Cells to cDNA kit (Ambion) according to the manufacturer's protocol. IL-1β transcript levels were then measured using Taqman primer/probe assay sets (Applied Biosystems), normalized to GAPDH using the deltaCt method, and the data expressed relative to vehicle only control. Figure 1 summarizes the IL-1β gene expression data on compound **I-57** (cellular toxicity was assessed using alamar blue as indicator, abbreviated in the figure as AB). As shown in this figure, upon dosing with compound **I-57**, there was a decrease in the gene expression of the inflammatory cytokine IL-1β.

### Example 10

### Effect of fatty acid amides on the target gene Hmox1 in RAW macrophages

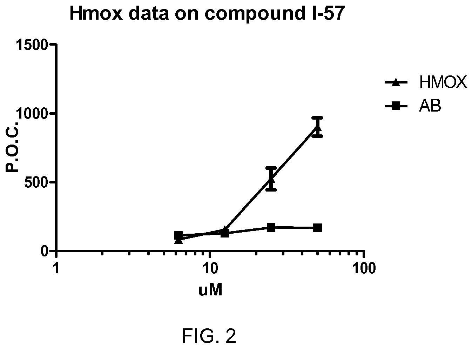

RAW264.7 macrophages were seeded at a density of 100,000 cells/well in a 96-well plate in DMEM supplemented with 10% FBS and Penn/strep. 16 hours later, medium was aspirated and replaced with 90uL/well of serum-free DMEM. Fatty acid amides were brought up in 100% EtOH to a concentration of 100mM and then diluted 1:100 in 100% FBS for a 20x stock solution consisting of ImM compound and 1% EtOH. The fatty acid amide 20x stock solutions were diluted 1:2 in FBS supplemented with 1% EtOH for a 500uM 10x stock solution. The 10x stock solutions were then serially diluted 1:2 in FBS supplemented with 1% EtOH and 10µL of each dilution was added to the RAW246.7 cells to generate final concentrations of 50, 25, 12.5, 6.25, 3.12 and 1.6 µM. The compounds were allowed to pre-incubate for 2 hours before stimulation of 100ng/ml LPS (10µL of 1µg/ml LPS is added to each well). Following 3 hours of LPS stimulation, cells were washed once in 1x PBS, aspirated dry, and flash frozen in liquid nitrogen. RNA was then isolated and converted to cDNA using the Cells to cDNA kit (Ambion) according to the manufacturer's protocol. Transcript levels were then measured using ABI Taqman primer/probe assay kits, normalized to GAPDH using the deltaCt method, and the data expressed relative to vehicle only control. Figure 2 summarizes the Hmox data on compound **I-57** (cellular toxicity was assessed using alamar blue as indicator, abbreviated in the figure as AB). As shown in this figure, upon dosing with compound **I-57,** there was an upregulation of Hmox gene expression.

### Example 11

### Effect of fatty acid amides conjugates in the streptozotocin-diabetic rat

Female Sprague-Dawley rats (8 weeks old, with an average weight of 150 g) are used for the study. Diabetes is induced by a single tail vein injection of streptozotocin (STZ) in 0.1 mol/L sodium citrate buffer, pH 4.5. Diabetes is then confirmed by measuring blood glucose levels at two and three days after the STZ treatment. Diabetic animals are classified as those with plasma glucose higher than 16 nmol/L. The diabetic animals are then divided into the vehicle control group and the treatment group (each group having 12 animals). All animals are housed individually with a light dark cycle of 12 hours each, with animals having free access to food and water. In order to maintain body weight and to limit hyperglycemia, diabetic animals are treated with 3 IU of ultralente insulin three times per week in the afternoon (at approximately 3 to 4 pm). In order to maintain glycemic control as the animals gain weight, the dose of insulin is increased to 5 IU at week 15. Animals are dosed with the vehicle or the fatty acid amide over a 28 week period (Examples of vehicles that can be used include combinations of solvents such as polyethylene glycol and propyleneglycol, lipids such as glycerol monooleate and soybean oil, and surfactants such as polysorbate 80 and cremophor EL). Progression of renal disease can be assessed by monthly measurements of urinary albumin and plasma creatinine concentrations. For urinary measurements, rats are housed in metabolic rat cages for 24 hrs. Urinary albumin can be quantified by a competitive ELISA assay according to the protocols outlined in Degenhardt et al, Kidney International 2002, 61, p. 939-950. Plasma creatinine concentrations can be measured by the Jaffé picric acid procedure, using the standard kit from Sigma (Sigma cat # 555-A). Statistical analyses can be performed using SigmaStat for Windows V1.00. *P* values can be calculated by non-parametric Mann-Whitney Rank Sum analysis. On week 28, dyslipidemia can also be assessed by measuring plasma triglycerides and total cholesterol. These plasma lipids can be measured by enzymatic, colorimetric, end-point assays using standardized, commercially available kits. Total cholesterol can be analyzed using the Sigma kit (cat # 352) and triglycerides can be analyzed by the Sigma kit (cat # 37, GOP Grinder).

### Example 12

### Effect of fatty acid amides in the cisplatin-induced nephrotoxicity mouse model

For this study, 10 to 12-week old male C57BL/6 mice of approximately 30 g in body weight are used. After the normal acclimation period, the animals are maintained on a standard diet and water is freely available. Mice are then given a single intraperitoneal injection of either the vehicle or cisplatin (20 mg/kg, at a concentration of 1 mg/mL in saline). Ten animals are used per treatment group. For the drug treatment group, beginning 24 hours prior to the cisplatin injection, animals are dosed with a fatty acid amide (formulated in combinations of solvents such as polyethylene glycol and propyleneglycol, lipids such as glycerol monooleate and soybean oil, and surfactants such as polysorbate 80 and cremophor EL). Dosing is then continued over a period of 72 hours. At this point, animals are sacrificed and blood and kidney tissues are collected. Blood urea nitrogen (BUN) and creatinine are measured. Levels of TNF-α in serum can be determined using a commercially available enzyme-linked immunosorbent assay (ELISA). Tissues are processed for histology and RNA isolation. Tubular injury can be assessed in PAS-stained sections using a semi-quantitative scale described in "G. Ramesh and W. B. Reeves, Kidney International, 2004, 65, p. 490-498".

### Compounds

The following non-limiting compound examples serve to illustrate further embodiments of the fatty acid amides.

### Example 13

### Preparation of (5Z,8Z,11Z,14Z,17Z)-N-(2-(2-(2-methoxyethoxy)ethoxy)ethyl)icosa-5,8,11,14,17-pentaenamide (I-57):

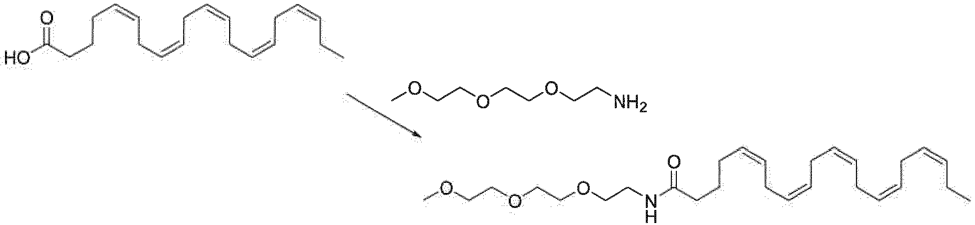

In a typical run, (5Z,8Z,11Z,14Z,17Z)-eicosa-5,8,11,14,17-pentaenoic acid (1 mmol) was taken up in 50 mL of CH₂Cl₂ along with 2-(2-(2-methoxyethoxy)ethoxy)ethanamine (1 mmol) and EDC (1.1 mmol). The resulting reaction mixture is stirred at room temperature for 6 h and then diluted with CH₂Cl₂ (100 mL). The organic layer is washed with saturated aqueous NH₄Cl, brine, dried (Na₂SO₄) and concentrated under reduced pressure. The resulting residue was purified by chromatography (95% CH₂Cl₂, 5% MeOH) to afford (5Z,8Z,11Z,14Z,17Z)-N-(2-(2-(2-methoxyethoxy)ethoxy)ethyl)icosa-5,8,11,14,17-pentaenamide. MS (EI) calcd for C₂₇H₄₅NO₄ 447.33; found 448 (M + 1).

### Example 14

### Preparation of (4Z,7Z,10Z,13Z,16Z,19Z)-N-(2-(2-(2-(2-methoxyethoxy)ethoxy)ethoxy)ethyl)docosa-4,7,10,13,16,19-hexaenamide (I-53):

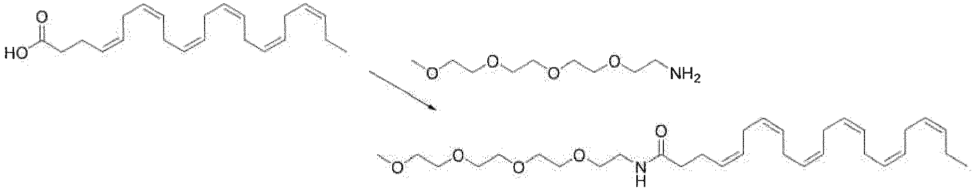

The same procedure outlined in example 13 was used. (4Z,7Z,10Z,13Z,16Z,19Z)-Docosa-4,7,10,13,16,19-hexaenoic acid and 2-(2-(2-(2-methoxyethoxy)ethoxy)ethoxy)ethanamine were used as the appropriate starting materials for the amide coupling step. MS (EI) calcd for C₃₁H₅₁NO₅ 517.38; found 518 [M+H]⁺.

### Example 15

### Preparation of (4Z,7Z,10Z,13Z,16Z,19Z)-N-(2-((2-acetamidoethyl)disulfanyl)ethyl)docosa-4,7,10,13,16,19-hexaenamide (I-2):

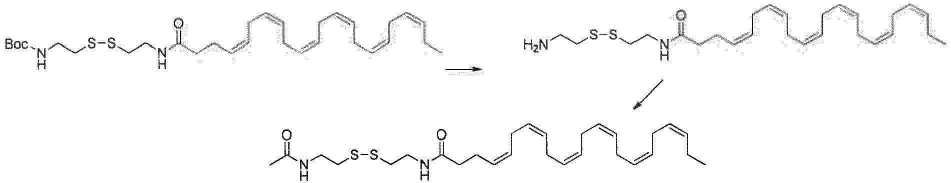

The starting material, tert-butyl (2-((2-((4Z,7Z,10Z,13Z,16Z,19Z)-docosa-4,7,10,13,16,19-hexaenamido)ethyl)disulfanyl)ethyl)carbamate, was prepared according to the procedures outlined in WO 2011106688.

To the solution of tert-butyl (2-((2-((4Z,7Z,10Z,13Z,16Z,19Z)-docosa-4,7,10,13,16,19-hexaenamido)ethyl)disulfanyl)ethyl)carbamate (10 g, 17.8 mmol) in 20 mL of EtOAc was added a 4 N HCl solution in EtOAc (100 mL) at 0 °C. The resulting reaction mixture was stirred at room temperature for 2 h. A saturated aqueous solution of Na₂CO₃ was added to the stirred mixture in order to adjust the pH is 8. The organic layer was separated and evaporated under reduced pressure to afford 8.2 g of (4Z,7Z,10Z,13Z,16Z,19Z)-N-(2-((2-aminoethyl)disulfanyl)ethyl)docosa-4,7,10,13,16,19-hexaenamide. Yield: 100%.

MS calculated for C₂₆H₄₂N₂OS₂: 462.75; Found: 463.5 [M+H]⁺.

A mixture of (4Z,7Z,10Z,13Z,16Z,19Z)-N-(2-((2-acetamidoethyl)disulfanyl)ethyl)docosa-4,7,10,13,16,19-hexaenamide (8.2 g, 17.8 mmol) in CH₂Cl₂ (200 mL) was cooled to 0 °C, and acetic anhydride (2.2 g, 21.3 mmol) and triethylamine (5.4 g, 53.4 mmol) was added. The resulting reaction mixture was allowed to slowly warm to room temperature over a period of 30 min under N₂. The organic layer was then washed with aq. NH₄Cl (100 mL x 3), brine (100 mL x 3), dried over anhydrous Na₂SO₄ and concentrated under reduced pressure. The resulting residue was purified by silica gel chromatography to afford 5.7 g of (4Z,7Z,10Z,13Z,16Z,19Z)-N-(2-((2-acetamidoethyl)disulfanyl)ethyl)docosa-4,7,10,13,16,19-hexaenamide. Yield: 64%.

MS calculated for C₂₈H₄₄N₂O₂S₂: 504.8; found: 505.2 [M+H]⁺.
¹H NMR (400 MHz, CDCl₃) δ 0.95-1.00 (t, *J* is 7.4 Hz, 3H), 2.02 (s,3H), 2.03-2.10 (m, 2H), 2.25-2.29 (m, 2H), 2.39-2.45 (m, 2H), 2.78-2.85 (m, 14H), 3.54-3.60 (m, 4H), 5.29-5.44 (m, 12H), 6.25 (s, 1H), 6.41 (s, 1H).

### Example 16

### Preparation of (5Z,8Z,11Z,14Z,17Z)-N-(2-((2-acetamidoethyl)disulfanyl)ethyl)icosa-5,8,11,14,17-pentaenamide (1-9):

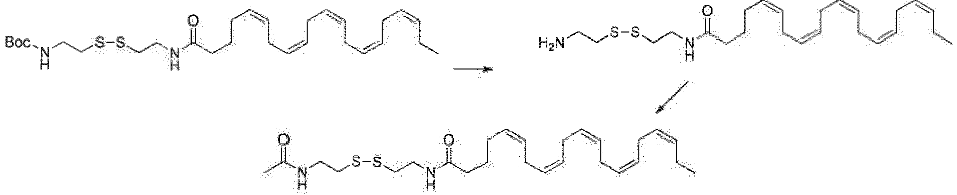

The starting material, tert-butyl (2-((2-((5Z,8Z,11Z,14Z,17Z)-icosa-5,8,11,14,17-pentaenamido)ethyl)disulfanyl)ethyl)carbamate was prepared according to the procedures outlined in WO 2011106688.

This material was then subjected to the same reaction sequence outlined in example 15 to prepare (5Z,8Z,11Z,14Z,17Z)-N-(2-((2-aminoethyl)disulfanyl)ethyl)icosa-5,8,11,14,17-pentaenamide. This fatty acid/cystamine derivative, (5Z,8Z,11Z,14Z,17Z)-N-(2-((2-aminoethyl)disulfanyl)ethyl)icosa-5,8,11,14,17-pentaenamide (12 g, 27.5 mmol), was then subjected to the same reaction conditions outlined in example 15. The final product was purified by silica gel chromatography to afford 6.7 g of (5Z,8Z,11Z,14Z,17Z)-N-(2-((2-acetamidoethyl)disulfanyl)ethyl)icosa-5,8,11,14,17-pentaenamide. Yield: 51%.

MS calculated for C₂₆H₄₂N₂O₂S₂: 478.8; found: 479.3 [M+H]⁺.
¹H NMR (400 MHz, CDCl₃) δ 0.95-1.00 (t, *J* is 7.6 Hz, 3 H), 1.68-1.76 (m, 2 H),2.02 (s, 3 H), 2.04-2.15 (m, 4 H), 2.20-2.24 (m, 2 H), 2.80-2.85 (m, 12 H), 3.53-3.60 (m, 4 H), 5.30-5.42 (m, 10 H), 6.25 (s, 1 H), 6.48 (s, 1 H).

### Example 17

### Preparation of (5Z,8Z,11Z,14Z,17Z)-N-(2-((2-(2,3-dihydroxypropanamido)ethyl)disulfanyl)ethyl)icosa-5,8,11,14,17-pentaenamide (I-10):

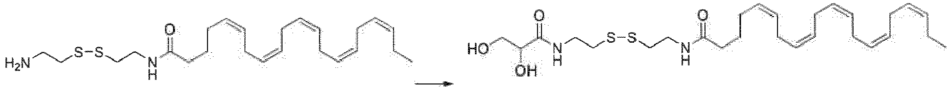

Glyceric acid was readily available commercially as an aqueous solution. The calcium salt of glyceric acid was isolated as follows: to a mixture of glyceric acid (20% in water) (50 g, 94.33 mmol) in H₂O (20 mL) was added Ca(OH)₂ (3.49 g, 47.16 mmol). The mixture was stirred at room temperature for 2 hours and then liophilized to afford 11.8 g of the calcium salt of glyceric acid.

The calcium salt of glyceric acid (4.81 g, 38.53 mmol) was taken up in 100 mL of DMF along with HOBt (6.5 g, 48.16 mmol), EDCI (9.23 g, 48.16 mmol), (5Z,8Z,11Z, 14Z,17Z)-N-(2-((2-aminoethyl)disulfanyl)ethyl)icosa-5,8,11,14,17-pentaenamide (14 g, 32.11 mmol) and DMAP (7.77 g, 64.22 mmol). The resulting reaction mixture was stirred at room temperature overnight. It was then diluted with ethyl acetate (500 mL) and washed with saturated aqueous NH₄Cl (100 mL x 3) and brine (100 mL x 3). The organic layer was dried over anhydrous Na₂SO₄ and concentrated under reduced pressure. The resulting residue was purified by silica gel chromatography to afford 3.4 g of (5Z,8Z,11Z,14Z,17Z)-N-(2-((2-(2,3-dihydroxypropanamido)ethyl)disulfanyl)ethyl)icosa-5,8,11,14,17-pentaenamide. Yield: 20 %.

MS calculated for C₂₇H₄₄N₂O₄S₂: 524.7; found: 525.3 [M+H]⁺.
¹H NMR (400 MHz, CDCl₃) δ 0.88-0.92 (t, *J* is 8 Hz, 3 H), 1.60-1.67 (m, 2 H), 1.97-2.15 (m, 6 H), 2.70-2.89 (m, 13 H), 3.43-3.57 (m, 4 H), 3.78-3.83 (m, 2 H), 4.11-4.23 (m, 2 H), 5.23-5.36 (m, 10 H), 6.04 (s, 1 H), 7.31 (s, 1 H).

### Example 18

### Preparation of 4-((2-((2-((5Z,8Z,11Z,14Z,17Z)-icosa-5,8,11,14,17-pentaenamido)ethyl)disulfanyl)ethyl)amino)-4-oxobutanoic acid (I-11):

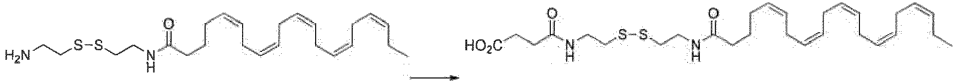

A mixture of (5Z,8Z,11Z,14Z,17Z)-N-(2-((2-aminoethyl)disulfanyl)ethyl)icosa-5,8,11,14,17-pentaenamide (14 g, 32.11 mmol) in CH₂Cl₂ (200 mL) was cooled down to 0 °C with stirring. Succinic anhydride (3.85 g, 38.53 mmol) and Et₃N (9.72 g, 96.33 mmol) were then added. The resulting reaction mixture was allowed to warm to room temperature and stirred for 18 h. It was then diluted with CH₂Cl₂ (200 mL) and acidified with some 1 N HCl (50 mL). The aqueous layer was discarded and the organic layer was washed with saturated NH₄Cl (300 mL x 3), brine (300 mL x 3), dried over anhydrous Na₂SO₄ and concentrated under reduced pressure. The resulting residue was purified by silica gel chromatography to afford 7.6 g of 4-((2-((2-((5Z,8Z,11Z,14Z,17Z)-icosa-5,8,11,14,17-pentaenamido)ethyl)disulfanyl)ethyl)amino)-4-oxobutanoic acid. Yield: 44%.

MS calculated for C₂₈H₄₄N₂O₄S₂: 536.7; found: 537.1 [M+H]⁺.
¹H NMR (400 MHz, CDCl₃) δ 0.88-0.92 (t, *J* is 7.6 Hz, 3 H), 1.62-1.68 (m, 2 H), 1.99-2.07 (m, 4 H), 2.13-2.17 (m, 2 H), 2.49-2.52 (m, 2 H), 2.62-2.64 (m, 2 H), 2.71-2.80 (m, 12 H), 3.47-3.52 (m, 4 H), 5.23-5.36 (m, 10 H), 6.13 (s, 1 H), 6.90 (s, 1 H).

### Example 19

### Preparation of 5-((2-((2-((5Z,8Z,11Z,14Z,17Z)-icosa-5,8,11,14,17-pentaenamido)ethyl)disulfanyl)ethyl)amino)-5-oxopentanoic acid (I-12):

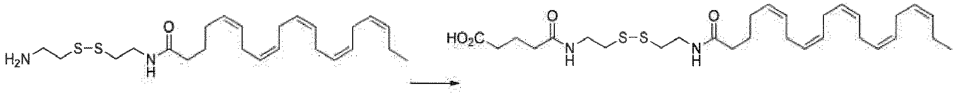

(5Z,8Z, 11Z, 14Z, 17Z)-N-(2-((2-Aminoethyl)disulfanyl)ethyl)icosa-5,8,11,14,17-pentaenamide (14 g, 32.11 mmol) was subjected to the same reaction conditions detailed in example 18, substituting glutaric anhydride for succinic anhydride. The resulting final product was purified by silica gel chromatography. Yield: 30%.

MS calculated for C₂₉H₄₆N₂O₄S₂: 550.8; found: 551.2 [M+H]⁺.
¹H NMR (400 MHz, CDCl₃) δ 0.88-0.92 (t, *J* is 7.6 Hz, 3 H), 1.62-1.68 (m, 2 H), 1.89-2.07 (m, 6 H), 2.13-2.17 (m, 2 H), 2.26-2.29 (m, 2 H), 2.34-2.38 (m, 2 H), 2.72-2.79 (m, 12 H), 3.47-3.52 (m, 4 H), 5.23-5.34 (m, 10 H), 6.16 (s, 1 H), 6.60 (s, 1 H).

### Example 20

### Preparation of (5Z,8Z,11Z,14Z,17Z)-N-(2-((2-((2R,3S,4R,5R)-2,3,4,5,6-pentahydroxyhexanamido)ethyl)disulfanyl)ethyl)icosa-5,8,11,14,17-pentaenamide (I-14)

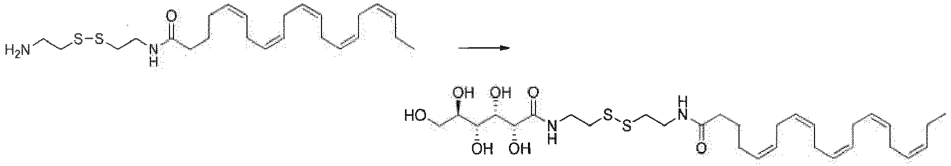

(5Z,8Z,11Z,14Z,17Z)-N-(2-((2-Aminoethyl)disulfanyl)ethyl)icosa-5,8,11,14,17-pentaenamide was subjected to the same reaction conditions detailed in example 17, substituting D-gluconic acid for glyceric acid. MS calculated for C₃₀H₅₀N₂O₇S₂: 614.31; found: 615 [M+H]⁺.

### Example 21

### Preparation of (4Z,7Z,10Z,13Z,16Z,19Z)-N-((S)-1-oxo-1-(((2R,3R,4R,5S,6R)-2,4,5-trihydroxy-6-(hydroxymethyl)tetrahydro-2H-pyran-3-yl)amino)propan-2-yl)docosa-4,7,10,13,16,19-hexaenamide (

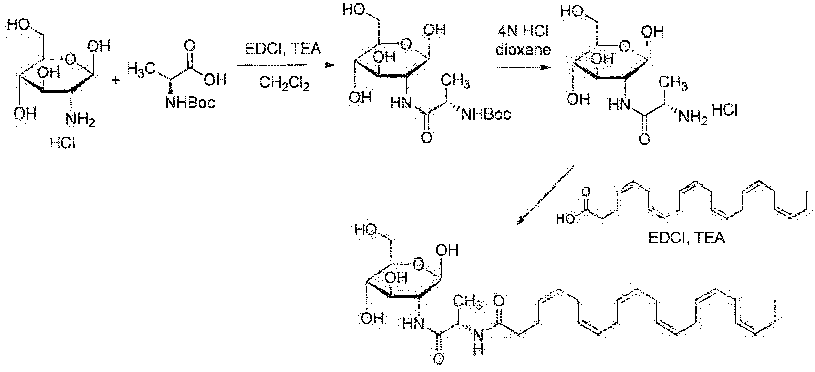

Glucosamine-HCl salt ((2R,3R,4R,5S,6R)-3-amino-6-(hydroxymethyl)tetrahydro-2H-pyran-2,4,5-triol hydrochloride, 500mg, 2.32 mmol) was pre-stirred in 3 mL CH₂Cl₂ and triethylamine (468 ul, 1.5 equivalents). Next, N-Boc-*L*-alanine ((S)-2-((tert-butoxycarbonyl)amino)propanoic acid, 439 mg, 1.0 equivalents) and EDC (672 mg, 1.5 equivalents) were added. The reaction was stirred at room temperature overnight under N₂ until completion. Upon completion, the crude reaction was filtered and washed with 2 mL CH₂Cl_{2;} the filtrate was dried under high vacuum to give an opaque waxy solid. The opaque solid was re-dissolved in hot EtOAC where white salt by-products remain undissolved. The heterogenous mixture was filtered, with EtOAc. The filtrate was collected, dried over Na₂SO₄, and dried under high vacuum to give tert-butyl ((S)-1-oxo-1-(((2R,3R,4R,5S,6R)-2,4,5-trihydroxy-6-(hydroxymethyl)tetrahydro-2H-pyran-3-yl)amino)propan-2-yl)carbamate, which was used in the next step without further purification.

To tert-butyl ((S)-1-oxo-1-(((2R,3R,4R,5S,6R)-2,4,5-trihydroxy-6-(hydroxymethyl)tetrahydro-2H-pyran-3-yl)amino)propan-2-yl)carbamate, was added 3 equivalents of 4N HCl in dioxane and stirred for 30 minutes, followed by azeotrope with EtOAc to give the (L)-alanine linked glucosamine-HCl salt, (S)-2-amino-N-((2R,3R,4R,5S,6R)-2,4,5-trihydroxy-6-(hydroxymethyl)tetrahydro-2H-pyran-3-yl)propanamide hydrochloride. The (L)-alanine linked glucosamine-HCl salt (605 mg, 2.11 mmol) was pre-stirred in 3 mL CH₂Cl₂ and triethylamine (426 ul, 1.5 equivalents). Next, (4Z,7Z,10Z,13Z,16Z,19Z)-docosa-4,7,10,13,16,19-hexaenoic acid (623 mg, 0.90 equivalents) and EDC (529 mg, 1.3 equivalents) were added and the reaction was stirred overnight at room temperature under N₂, until completion. Upon completion, the crude reaction was dried under high vacuum, then re-dissolved in EtOAC; next pentanes was added to form a 50% pentane-EtOAc mixture upon which salt-by products of the reaction crashes out. The crude product was filtered and the filtrate was concentrated under reduced pressure. The resulting residue was purified by silica gel chromatography (gradient elution, 0-10% MeOH in CH₂Cl₂). MS calculated for C₃₁H₄₈N₂O₇: 560.72; found 563.5 [M+Na]⁺.

### Example 22

### Preparation of (4Z,7Z,10Z,13Z,16Z,19Z)-N-((S)-5-((5Z,8Z,11Z,14Z,17Z)-icosa-5,8,11,14,17-pentaenamido)-6-oxo-6-(((2R,3R,4R,5S,6R)-2,4,5-trihydroxy-6-(hydroxymethyl)tetrahydro-2H-pyran-3-yl)amino)hexyl)docosa-4,7,10,13,16,19-hexaenamide (I-50):

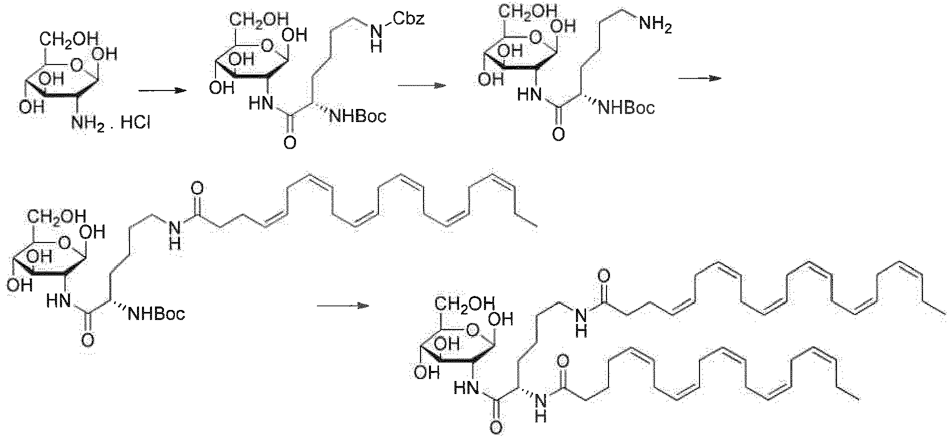

To a solution of (S)-benzyl tert-butyl (6-amino-6-oxohexane-1,5-diyl)dicarbamate (23 g, 60.52 mmol) in 150 mL of DMF was added successively Et₃N (18.31 g, 181.28 mmol), HATU (37.90 g 99.73 mmol), and glucosamine hydrochloride (14.33 g, 66.45 mmol) in portions at 0 °C. The resulting reaction mixture was stirred at room temperature for 2 hours and then concentrated under reduced pressure to remove the DMF. The resulting residue was purified by silica gel chromatography (gradient elution: CH₂Cl2/MeOH is 30:1 to 15:1) to afford 27 g of benzyl tert-butyl ((S)-6-oxo-6-(((2R,3R,4R,5S,6R)-2,4,5-trihydroxy-6-(hydroxymethyl)tetrahydro-2H-pyran-3-yl)amino)hexane-1,5-diyl)dicarbamate (Yield: 84%). MS calculated for C₂₅H₃₈N₂O₁₀: 540.58; found: 541.2 [M+H]⁺.

To a solution of benzyl tert-butyl ((S)-6-oxo-6-(((2R,3R,4R,5S,6R)-2,4,5-trihydroxy-6-(hydroxymethyl)tetrahydro-2H-pyran-3-yl)amino)hexane-1,5-diyl)dicarbamate (27 g, 49.9 mmol) in 300mL of methanol was added 10% Pd-C (3 g) and H₂ gas was introduced. The suspension was stirred at room temperature under 1 atm of H₂ for 18 h. The resulting reaction mixture was filtered through a pad of Celite and the filtrate was concentrated under reduced pressure to afford 20 g of tert-butyl ((S)-6-amino-1-oxo-1-(((2R,3R,4R,5S,6R)-2,4,5-trihydroxy-6-(hydroxymethyl)tetrahydro-2H-pyran-3-yl)amino)hexan-2-yl)carbamate (yield: 99%) as a yellow oil. This material was used for the next step without further purification. MS calculated for C₁₇H₃₂N₃O₈: 406.45; found: 407.1 [M+H]⁺.

To a solution of DHA (5.31 g, 16.21 mmol) in 100 mL of DMF was added Et₃N (2.23 g, 22.11 mmol), EDC (4.23 g, 22.11 mmol), then ((S)-6-amino-1-oxo-1-(((2R,3R,4R,5S,6R)-2,4,5-trihydroxy-6-(hydroxymethyl)tetrahydro-2H-pyran-3-yl)amino)hexan-2-yl)carbamate (6.0 g, 14.74 mmol) in portions at 0 °C. The reaction mixture was stirred at room temperature for 18 h and then concentrated under reduced pressure. The resulting residue was purified by silica gel chromatography (gradient elution: CH₂Cl₂: MeOH is 40:1 to 15:1) to afford 2.8 g of tert-butyl ((S)-6-((4Z,7Z,10Z,13Z,16Z,19Z)-docosa-4,7,10,13,16,19-hexaenamido)-1-oxo-1-(((2R,3R,4R,5S,6R)-2,4,5-trihydroxy-6-(hydroxymethyl)tetrahydro-2H-pyran-3-yl)amino)hexan-2-yl)carbamate (Yield: 26%).
MS calculated for C₃₉H₈₆₂N₃O₉: 716.9; found: 717.1 [M+H]⁺.

tert-Butyl ((S)-6-((4Z,7Z,10Z,13Z,16Z,19Z)-docosa-4,7,10,13,16,19-hexaenamido)-1-oxo-1-(((2R,3R,4R,5S,6R)-2,4,5-trihydroxy-6-(hydroxymethyl)tetrahydro-2H-pyran-3-yl)amino)hexan-2-yl)carbamate (2.8 g, 3.90 mmol) was taken up in 20 mL of 4 N HCl in ethyl acetate and allowed to stir at room temperature for 30 min. The reaction mixture was diluted with 20 mL of ethyl acetate and concentrated under reduced pressure to afford the HCl salt of (4Z,7Z,10Z,13Z,16Z,19Z)-N-((S)-5-amino-6-oxo-6-(((2R,3R,4R,5S,6R)-2,4,5-trihydroxy-6-(hydroxymethyl)tetrahydro-2H-pyran-3-yl)amino)hexyl)docosa-4,7,10,13,16,19-hexaenamide.

To a solution of compound EPA (1.29 g,4.29 mmol) in 50 mL of DMF was added Et₃N (0.59 g, 5.85 mmol), HATU (2.22 g , 0.85 mmol), and the HCl salt of (4Z,7Z,10Z, 13Z, 16Z, 19Z)-N-((S)-5-amino-6-oxo-6-(((2R,3R,4R,5S,6R)-2,4,5-trihydroxy-6-(hydroxymethyl)tetrahydro-2H-pyran-3-yl)amino)hexyl)docosa-4, 7,10,13,16,19-hexaenamide (2.54 g, 3.90 mmol) in portions at 0 °C. The resulting reaction mixture was stirred at room temperature overnight. Then the reaction mixture was removed under reduced pressure. The residue was purified by silica gel chromatography (gradient elution: CH₂Cl₂: MeOH is 50:1 to 15:1) to afford 120 mg of (4Z,7Z,10Z,13Z,16Z,19Z)-N-((S)-5-((5Z,8Z, 11Z, 14Z, 17Z)-icosa-5,8, 11,14, 17-pentaenamido)-6-oxo-6-(((2R,3R,4R,5S,6R)-2,4,5-trihydroxy-6-(hydroxymethyl)tetrahydro-2H-pyran-3-yl)amino)hexyl)docosa-4,7,10,13,16,19-hexaenamide (Yield: 3.1%).

MS calculated for C₅₄H₈₃N₃O₈: 902.25; found: 884 [M-18]⁺.
¹H NMR (400MHz, CDCl₃) δ 0.86-0.93 (m, 6H), 1.20-1.35 (m, 7H), 1.51-1.58 (m, 3H), 1.99-2.26 (m,12H), 2.76-2.81 (m, 18H), 2.95-2.98 (m, 3H), 3.47-3.59 (m, 3H), 4.39-4.42 (m, 2H), 4.57-4.59 (m, 1H), 4.90-4.91 (m,1H), 5.26-5.39 (m, 22H), 6.41-6.42 (m, 1H), 7.59-7.61 (m,1H), 7.72-7.73 (m, 1H), 7.81-7.83 (m, 1II).

### Example 23

### Preparation of (4Z,7Z,10Z,13Z,16Z,19Z)-N-((R)-6-(propylamino)-4,5,6,7-tetrahydrobenzo[d]thiazol-2-yl)docosa-4,7,10,13,16,19-hexaenamide (I-21):

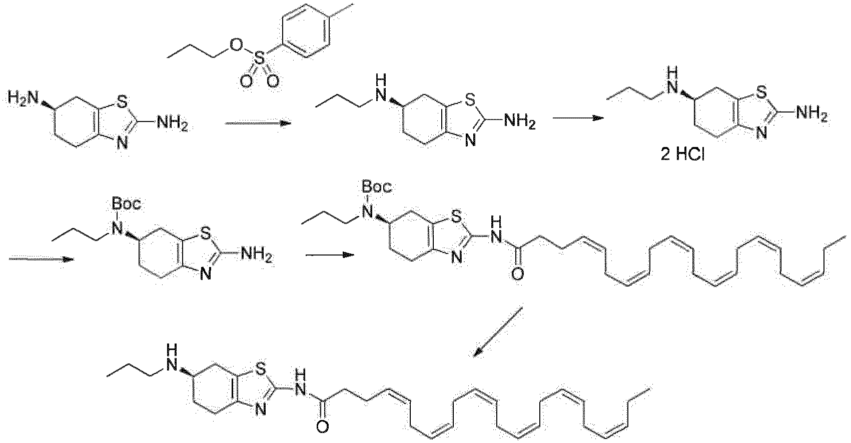

A mixture of the commercially available compound (R)-4,5,6,7-tetrahydrobenzo[d]thiazole-2,6-diamine (61 g, 351 mmol) in n-propanol (800 mL) was stirred at 95 °C under N₂ for 15 min. A solution containing n-propyl 4-methylbenzenesulfonate (100 g, 467 mmol) and DIEA (80 ml) in 300ml n-propanol was then added dropwise at room temperature. The resulting reaction mixture was stirred at 95 °C for 18 h. It was then cooled to room temperature, and stirring was continued for an additional 4 h. The precipitated material was collected by filtration and washed with reagent grade alcohol (120 mL), followed by heptanes (100 mL). The resulting solids were dried under high vacuum for 2 h and then taken up in 50 mL of ethanol and cooled to between 0 and 5 °C. With continuous stirring, concentrated HCl (45 mL) was slowly added to the reaction while maintaining the temperature at between 0 and 5 °C, and the mixture was stirred for an additional 15 min. Methyl t-butyl ether (MTBE, 270 mL) was added to mixture, and stirring was continued for additional 1.5 h at this temperature. The mixture was filtered, washed twice with an MTBE /ethanol solution (2:1; 2×70 mL), and dried under high vacuum at room temperature for 18 h to afford 38.5 g of the di-hydrochloride salt of (R)-N6-propyl-4,5,6,7-tetrahydrobenzo[d]thiazole-2,6-diamine (Yield: 51%). MS calculated for C₁₀H₁₇N₃S: 211.11; found: 212.1 [M+H]⁺.

The di-hydrochloride salt of (R)-N6-propyl-4,5,6,7-tetrahydrobenzo[d]thiazole-2,6-diamine (12 g, 42.2 mmol) and Et₃N (12.7 g, 126.6 mmol) were taken up in 500 mL of MeOH. A solution of Boc-anhydride (79.14 g, 0.665 mmol) in MeOH (70 mL) was then added dropwise at room temperature over a period of about 1 h. The resulting reaction mixture was stirred at room temperature for 18 h and then concentrated under reduced pressure. It was then diluted with water (100 mL) and extracted with CH₂Cl₂. The combined organic layers were dried over anhydrous Na₂SO₄ and concentrated under reduced pressure. The resulting residue was purified by silica gel chromatography (using a mixture of pentane/EtOAc) to afford 6.3 g of (R)-tert-butyl (2-amino-4,5,6,7-tetrahydrobenzo[d]thiazol-6-yl)(propyl)carbamate (Yield: 49%).

(R)-tert-Butyl (2-amino-4,5,6,7-tetrahydrobenzo[d]thiazol-6-yl)(propyl)carbamate (4 g, 12.8 mmol) was taken up in 100 mL of CH₂Cl₂ along with DHA (4 g, 12.2 mmol), HATU (5.9 g, 15.4 mmol) and DIEA (4.96 g, 38.4 mmol). The resulting reaction mixture was stirred at room temperature for 18 h. It was then diluted with CH₂Cl₂ (100 mL) and washed with aq. HCl (5%, 3 x 200 mL) and brine (3 x 200 mL). The organic layer was dried over anhydrous Na₂SO₄ and concentrated under reduced pressure. The resulting residue was purified by silica gel chromatography (pentane/EtOAc) to afford 5 g of tert-butyl ((R)-2-((4Z,7Z, 10Z, 13Z, 16Z, 19Z)-docosa-4,7,10, 13,16,19-hexaenamido)-4,5,6,7-tetrahydrobenzo[d]thiazol-6-yl)(propyl)carbamate (Yield: 66%).

MS calculated for C₃₇H₅₅N₃O₃S: 621.9; found: 622.4 [M+H]⁺.
¹H NMR (400 MHz, CDCl₃) δ 0.87-0.91 (t, *J* is 7.4 Hz, 3H), 0.94-0.99 (t, *J* is 7.4 Hz, 3H), 1.47 (s, 9H), 1.52-1.63 (m, 2H), 1.82-2.11 (m, 4H), 2.40-2.48 (m, 4H), 2.72-2.77 (m, 20H), 3.00-3.10 (m, 2H), 5.27-5.45 (m, 12H).

tert-Butyl ((R)-2-((4Z,7Z, 10Z, 13Z, 16Z, 19Z)-docosa-4,7, 10,13, 16, 19-hexaenamido)-4,5,6,7-tetrahydrobenzo[d]thiazol-6-yl)(propyl)carbamate (2.1 g, 3.43 mmol) was taken up in 20 mL of 4 N HCl in ethyl acetate and allowed to stir at room temperature for 2 h. The reaction mixture was diluted with 40 mL of ethyl acetate and concentrated under reduced pressure. The resulting residue was diluted with EtOAc and washed with aq. NaHCO₃ (3 x 30 mL) brine (3 x 30 mL). The organic layer was dried over anhydrous Na₂SO₄ and concentrated under reduced pressure. The resulting residue was purified by preparative HPLC to afford 1.5 g of (4Z,7Z,10Z,13Z,16Z,19Z)-N-((R)-6-(propylamino)-4,5,6,7-tetrahydrobenzo[d]thiazol-2-yl)docosa-4,7,10,13,16,19-hexaenamide (Yield: 85%).

MS calculated for C₃₂H₄₇N₃OS: 521.8; found: 522.3 [M+H]⁺.
1H NMR (400 MHz, CDCl₃) δ 0.92-0.99 (m, 6H), 1.50-1.56 (m, 2H), 1.72-1.74 (m, 2H), 2.03-2.09 (m, 4H), 2.46-2.51 (m, 5H), 2.64-2.73 (m, 3H), 2.79-2.85 (m, 11H), 2.97-3.02 (m, 2H), 5.32-5.43 (m, 12H).

### Example 24

### Preparation of (5Z,8Z,11Z,14Z,17Z)-N-((R)-6-(propylamino)-4,5,6,7-tetrahdrobenzodthiazol-2-yl)icosa-5,8,11,14,17-pentaenamide (I-22):

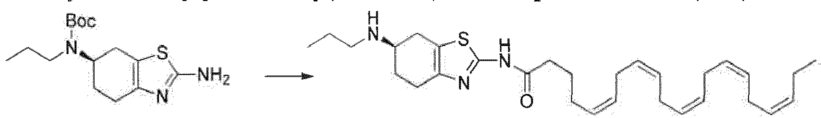

(R)-tert-Butyl (2-amino-4,5,6,7-tetrahydrobenzo[d]thiazol-6-yl)(propyl)carbamate (300 mg, 0.96 mmol) was subjected to the same reaction conditions detailed in example 23, substituting EPA for DHA. After preparative HPLC purification, 180 mg of (5Z,8Z,11Z, 14Z, 17Z)-N-((R)-6-(propylamino)-4,5,6,7-tetrahydrobenzo[d]thiazol-2-yl)icosa-5,8,11,14,17-pentaenamide.

MS calculated for C₃₀H₄₅N₃OS: 495.33; found: 496.3 [M+H]⁺.
1H NMR (400 MHz, CDCl₃) δ 0.98-1.04 (m, 6H), 1.50-1.70 (s, 3H), 1.71-1.84 (m, 3H), 2.03-2.07 (m, 3H), 2.12-2.18 (m, 2H), 2.39-2.43 (t, *J* is 3.6 Hz, 3H), 2.49-2.51 (m, 1H), 2.64-2.67 (t, *J* is 7.4 Hz, 3H), 2.71-2.74 (m, 8H), 2.95-3.03 (m, 2H), 5.29-5.43 (m, 10H).

### Example 25

### Preparation of (4Z,7Z,10Z,13Z,16Z,19Z)-N-(3-oxo-3-(((R)-6-(propylamino)-4,5,6,7-tetrahydrobenzo[d]thiazol-2-yl)amino)propyl)docosa-4,7,10,13,16,19-hexaenamide (I-27):

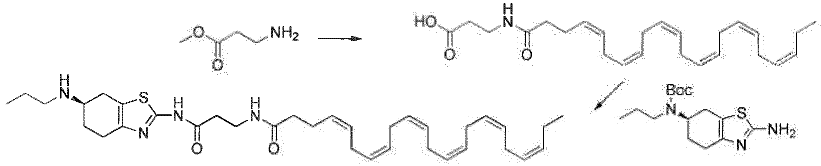

To a suspension of β-alanine methyl ester (1.39 g, 10 mmol), EDC (2.88 g, 15 mmol), HOBt (2.03 g, 15 mmol) and Et₃N (3.03 g, 30 mmol) in 30 mL of CH₂Cl₂ was added DHA (3.12 g, 9.5 mmol). The mixture was stirred at room temperature for 18 h. The reaction mixture was then washed with saturated aqueous NH₄Cl (3 x 30 mL), brine (3 x 30 mL), dried over anhydrous Na₂SO₄ and concentrated under reduced pressure. The resulting residue was purified by silica gel chromatography (EtOAc/pentane) to afford 3.2 g of methyl 3-((4Z,7Z, 10Z, 13Z, 16Z, 19Z)-docosa-4,7, 10, 13, 16, 19-hexaenamido)propanoate (Yield: 82%).
¹H NMR (400 MHz, CDCl₃) δ 0.95-0.99 (t, *J* is 7.6 Hz, 3H), 2.03-2.11 (m, 2H), 2.21-2.25 (t, *J* is 7.6 Hz, 2H), 2.37-2.43 (m, 2H), 2.52-2.56 (t, *J* is 6.0 Hz, 2H), 2.79-2.86 (m, 10H), 2.37-2.43 (m, 2H), 3.70 (s, 3H), 5.29-5.42 (m, 12H), 6.06 (s, 1H).

To a solution of methyl 3-((4Z,7Z,10Z,13Z,16Z,19Z)-docosa-4,7,10,13,16,19-hexaenamido)propanoate (0.7 g, 1.69 mmol) in 10 mL of THF was added 10 mL of 5 N NaOH. The resulting reaction mixture was stirred at room temperature for 2h and acidified to pH is 2 with 6 N HCl. The aqueous phase was extracted with EtOAc. The combined organic layers were washed with brine, dried over Na₂SO₄ and concentrated under reduced pressure. The resulting residue was purified by silica gel chromatography to afford 532 mg of 3-((4Z,7Z,10Z, 13Z, 16Z, 19Z)-docosa-4,7,10,13,16,19-hexaenamido)propanoic acid (Yield: 80%).
¹H NMR (400 MHz, CDCl₃) δ 0.95-0.99 (t, *J* is 7.4 Hz, 3H), 2.03-2.08 (m, 2H), 2.21-2.25 (t, *J* is 7.2 Hz, 2H), 2.37-2.43 (m, 2H), 2.58-2.62 (t, *J* is 5.4 Hz, 2H), 2.79-2.90 (m, 10H), 3.52-3.54 (3, 2H), 5.29-5.43 (m, 12H), 6.08 (s, 1H).

3-((4Z,7Z,10Z,13Z,16Z,19Z)-Docosa-4,7,10,13,16,19-hexaenamido)propanoic acid (200 mg, 0.50 mmol) was taken up in 10 mL of CH₂Cl₂ along with (R)-tert-butyl (2-amino-4,5,6,7-tetrahydrobenzo[d]thiazol-6-yl)(propyl)carbamate (156 mg, 0.50 mmol), HATU (229 mg, 0.60 mmol) and DIEA (194 mg, 1.50 mmol). The resulting reaction mixture was stirred at room temperature for 18 h. It was then diluted with CH₂Cl₂ (10 mL) and washed with aq. HCl (5%, 3 x 20 mL) and brine (3 x 20 mL). The organic layer was dried over anhydrous Na₂SO₄ and concentrated under reduced pressure. The resulting residue was purified by silica gel chromatography (EtOAc/pentane) to afford 200 mg of tert-butyl ((R)-2-(3-((4Z,7Z,10Z,13Z,16Z,19Z)-docosa-4,7,10,13,16,19-hexaenamido)propanamido)-4,5,6,7-tetrahydrobenzo[d]thiazol-6-yl)(propyl)carbamate (Yield: 57%).

tert-Butyl ((R)-2-(3-((4Z,7Z,10Z, 13Z, 16Z, 19Z)-docosa-4,7,10,13,16,19-hexaenamido) propanamido)-4,5,6,7-tetrahydrobenzo[d]thiazol-6-yl)(propyl)carbamate (200 mg, 0.29 mmol) was taken up in 10 mL of 4 N HCl in EtOAc and allowed to stir at room temperature for 2 h. The reaction mixture was diluted with 10 mL of EtOAc and concentrated under reduced pressure. The resulting residue was purified by preparative HPLC to afford 120 mg of (4Z,7Z,10Z,13Z,16Z,19Z)-N-(3-oxo-3-(((R)-6-(propylamino)-4,5,6,7-tetrahydrobenzo[d] thiazol-2-yl)amino)propyl)docosa-4,7,10,13,16,19-hexaenamide (Yield: 70%)

MS calculated for C₃₅H₅₂N₄O₂S: 592.9; found: 593.4 [M+H]⁺.
¹H NMR (400 MHz, CDCl₃) δ 0.88-0.97 (m, 6H), 1.50-1.56 (m, 2H), 1.71-1.82 (m, 2H), 2.00-2.09 (m, 4H), 2.20-2.24 (m, 2H), 2.36-2.40 (m, 2H), 2.41-2.60 (m, 1H), 2.65-2.68 (m, 5H), 2.69-2.73 (m, 1H), 2.80-2.86 (m, 10H), 2.95-3.03 (m, 2H), 3.58-3.63 (m, 2H), 5.30-5.42 (m, 12H), 6.22-5.24 (m, 1H).

### Example 26

### Preparation of (4Z,4'Z,7Z,7'Z,10Z,10'Z,13Z,13'Z,16Z,16'Z,19Z,19'Z)-N,N'-((S)-6-oxo-6-(((R)-6-(propylamino)-4,5,6,7-tetrahydrobenzo[d]thiazol-2-yl)amino)hexane-1,5-diyl)bis(docosa-4,7,10,13,16,19-hexaenamide) (I-82):

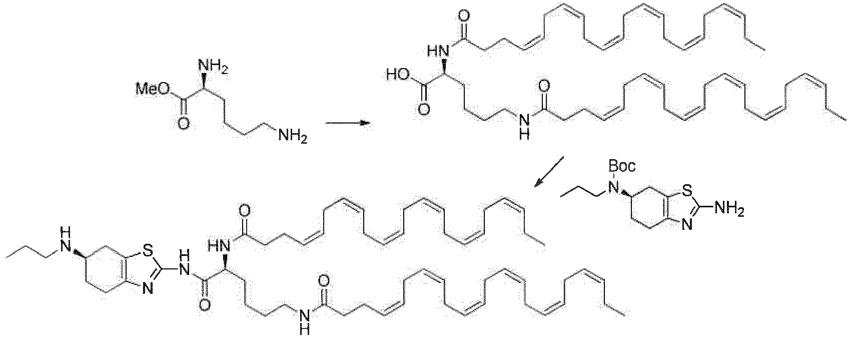

To a suspension of L-lysine methyl ester dihydrochloride (2.33 g, 10 mmol), EDC (2.88 g, 15 mmol), HOBt (2.03 g, 15 mmol) and Et₃N (3.03 g, 30 mmol) in 30 mL of CH₂Cl₂ was added DHA (3.12 g, 9.5 mmol). The resulting reaction mixture was stirred at room temperature for 18 h. The reaction mixture was washed with saturated aqueous NH₄Cl (3 x 30 mL) and brine (3 x 30 mL). The organic layer was dried over anhydrous Na₂SO₄ and concentrated under reduced pressure. The resulting residue was purified by silica gel chromatography (pentane/EtOAc) to afford 4.67 g of (S)-methyl 2,6-di((4Z,7Z,10Z,13Z,16Z,19Z)-docosa-4,7,10,13,16,19-hexaenamido)hexanoate (Yield: 60%). ¹H NMR (400 MHz, CDCl₃) δ 0.95 -1.01 (m, 6H), 1.26 - 1.40 (m, 2H), 1.48 - 1.55 (m, 2H), 1.65 - 1.73 (m, 1H), 1.79 - 1.85 (m, 2H), 2.03 - 2.13 (m, 4H), 2.19 - 2.24 (m, 2H), 2.27 - 2.32 (m, 2H), 2.37 - 2.45 (m, 4H), 2.80 - 2.85 (m, 20H), 3.20 - 3.27 (m, 2H), 3.74 - 3.75 (d, *J* is 2.4 Hz, 3H), 4.56 - 4.60 (m, 1H), 5.27 - 5.46 (m, 24H), 5.68 (s, 1H), 6.18 - 6.21 (d, *J* is 7.5 Hz, 1H).

To the solution of (S)-methyl 2,6-di((4Z,7Z,10Z,13Z,16Z,19Z)-docosa-4,7,10,13,16,19-hexaenamido)hexanoate (2.5 g, 3.2 mmol) in 20 mL of THF was added 50 mL of 5 N NaOH. The resulting reaction mixture was stirred at room temperature for 2h and then acidified to pH is 2 with 6 N HCl. The aqueous phase was extracted with EtOAc. The combined organic layers were washed with brine, dried over Na₂SO₄, and concentrated under reduced pressure to afford 2.22 g of (S)-2,6-di((4Z,7Z,10Z,13Z,16Z,19Z)-docosa-4,7,10,13,16,19-hexaenamido)hexanoic acid (Yield: 90%).

(S)-2,6-di((4Z,7Z,10Z,13Z,16Z,19Z)-Docosa-4,7,10,13,16,19-hexaenamido)hexanoic acid (500 mg, 0.70 mmol) was taken up in 20 mL of CH₂Cl₂ along with (R)-tert-butyl (2-amino-4,5,6,7-tetrahydrobenzo[d]thiazol-6-yl)(propyl)carbamate (204 mg, 0.70 mmol), HATU (319 mg, 0.84 mmol) and DIEA (226 mg, 1.75 mmol). The resulting reaction mixture was stirred at room temperature for 18 h. It was then diluted with CH₂Cl₂ (20 mL) and washed with aq. HCl (5%, 3 x40 mL) and brine (3 x 40 mL). The organic layer was dried over anhydrous Na₂SO₄ and concentrated under reduced pressure. The resulting residue was purified by silica gel chromatography (pentane/EtOAc) to afford 300 mg of tert-butyl ((R)-2-((S)-2,6-di((4Z,7Z,10Z,13Z,16Z,19Z)-docosa-4,7,10,13,16,19-hexaenamido)hexanamido)-4,5,6,7-tetrahydrobenzo[d]thiazol-6-yl)(propyl)carbamate (Yield: 72%).

tert-Butyl ((R)-2-((S)-2,6-di((4Z,7Z,10Z,13Z,16Z,19Z)-docosa-4,7,10,13,16,19-hexaenamido)hexanamido)-4,5,6,7-tetrahydrobenzo[d]thiazol-6-yl)(propyl)carbamate (500 mg, 0.47 mmol) was taken up in 20 mL of 4 N HCl in ethyl acetate and allowed to stir at room temperature for 90 min. The reaction mixture was diluted with 10 mL of ethyl acetate and concentrated under reduced pressure to afford 300 mg of (4Z,4'Z,7Z,7'Z,10Z,10'Z,13Z,13'Z,16Z,16'Z,19Z,19'Z)-N,N'-((S)-6-oxo-6-(((R)-6-(propylamino)-4,5,6,7-tetrahydrobenzo[d]thiazol-2-yl)amino)hexane-1,5-diyl)bis(docosa-4,7,10,13,16,19-hexaenamide) (Yield: 70%).

MS calculated for C₆₀H₈₉N₅O₃S: 960.4; found: 961.4 [M+H]⁺.
¹H NMR (400 MHz, CDCl₃) δ 0.94 - 0.99 (m, 9H), 1.40-1.46 (m, 2H), 1.50 - 1.54 (m, 2H), 1.61 - 1.67 (m, 2H), 1.74 -1.86 (m, 2H), 1.90 -1.95 (m, 2H), 2.03 - 2.10 (m, 4H), 2.20 - 2.29 (m, 3H), 2.32 - 2.43 (m, 7H), 2.46 - 2.66 (m, 2H), 2.74 - 2.84 (m, 23H), 3.01 - 3.05 (m, 1H), 3.17 - 3.32 (m, 3H), 4.59 - 4.62 (m, 1H), 5.27 - 5.44 (m, 24H), 5.80 - 5.82 (m, 1H), 6.74 - 6.76 (m, 1H).

### Example 27

### Preparation of (5Z,8Z,11Z,14Z,17Z)-N-((S)-4-methyl-1-oxo-1-(((R)-6-(propylamino)-4,5,6,7-tetrahydrobenzo[d]thiazol-2-yl)amino)pentan-2-yl)icosa-5,8,11,14,17-pentaenamide (I-31)

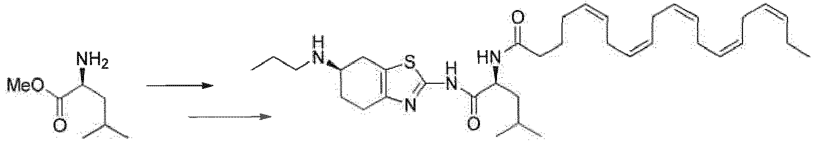

The same experimental procedure outlined in example 25 was employed, using L-Leucine methyl ester as the appropriate starting material.

MS calculated for C₃₉H₅₈N₄O₂S: 634.4; found: 635.4 [M+H]⁺.
¹H NMR (400 MHz, CDCl₃) δ 0.86 - 1.01 (m, 12H), 1.47 - 1.55 (m, 2H), 1.57 - 1.69 (m, 2H), 1.71 - 1.80 (m, 2H), 2.04 - 2.13 (m, 4H), 2.23 - 2.29 (m, 3H), 2.33 - 2.40 (m, 3H), 2.65 - 2.69 (t, *J* is 13.8 Hz, 3H), 2.82 - 2.87 (m, 10H), 2.96-3.04 (m, 2H), 4.66 - 4.73 (m, 1H), 5.30 - 5.45 (m, 12H), 5.95 - 5.98 (d, *J* is 7.5 Hz, 1H).

### Example 28

### Preparation of (5Z,8Z,11Z,14Z,17Z)-N-((S)-1-oxo-1-(((R)-6-(propylamino)-4,5,6,7-tetrahydrobenzo[d]thiazol-2-yl)amino)propan-2-yl)icosa-5,8,11,14,17-pentaenamide (I-29):

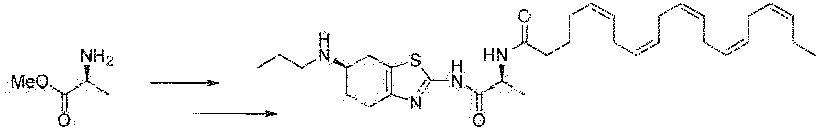

The same experimental procedure outlined in example 25 was employed, using L-Alanine methyl ester as the appropriate starting material.

MS calculated for C₃₅H₅₂N₄O₂S: 592.9; found: 593.4 [M+H]⁺.
¹H NMR (400 MHz, CDCl₃) δ 0.98-1.05 (m, 6H), 1.46-1.49 (d, *J* is 6.8 Hz, 3H),1.91-2.15 (m, 5H), 2.34-2.46 (m, 5H), 2.59-2.2.74 (m, 2H), 2.81-2.88 (m, 10H), 3.01-3.10 (m, 3H), 3.20-3.53 (m, 2H), 4.80-4.89 (m, 1H), 5.27-5.43 (m, 12H), 6.85-6.98 (m, 1H).

## Claims

1. A compound of the **Formula I:** 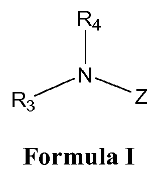 or a pharmaceutically acceptable salt, enantiomer or stereoisomer thereof;
wherein
Z is 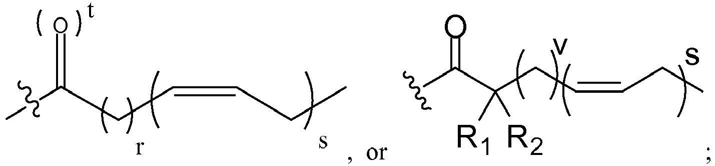
each r is independently 2, 3, or 7;
each s is independently 3, 5, or 6;
each t is independently 0 or 1;
each v is independently 1, 2, or 6;
R₁ and R₂ are independently -H, -D, -C₁-C₄ alkyl, -halogen, -OH, -C(O)C₁-C₄ alkyl, -O-aryl, -O-benzyl, -OC(O)C₁-C₄ alkyl, -C₂-C₃ alkene, -C₂-C₃ alkyne, -NH₂, -NH(C₁-C₃ alkyl), -N(C₁-C₃ alkyl)₂, -NH(C(O)C₁-C₃ alkyl), -N(C(O)C₁-C₃ alkyl)₂, -SH, -S(C₁-C₃ alkyl), -S(O)C₁-C₃ alkyl, or -S(O)₂C₁-C₃ alkyl;
R₃ is H
and R₄ is selected from the group consisting of 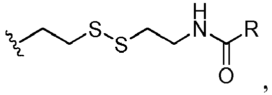 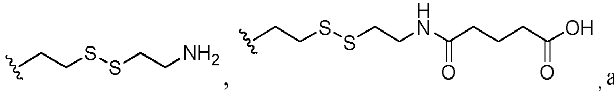 and 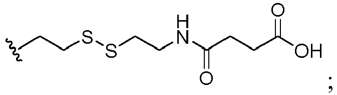 wherein
R is straight or branched unsubstituted -C₁-C₆ alkyl; or
a compound selected from 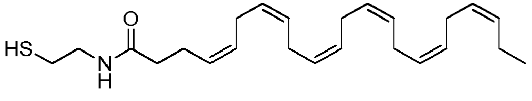 and 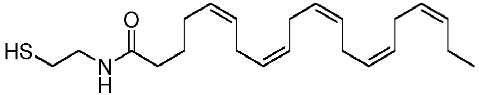 or a pharmaceutically acceptable salt thereof.

2. The compound of claim 1, wherein the compound is 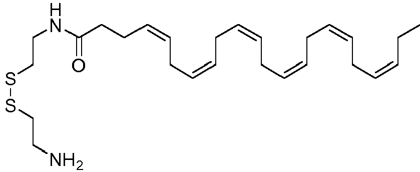 or a pharmaceutically acceptable salt thereof.

3. The compound of claim 1, wherein the compound is 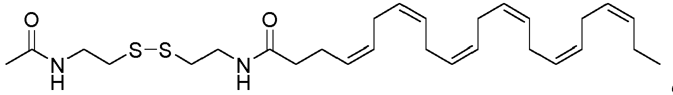 or a pharmaceutically acceptable salt thereof.

4. The compound of claim 1, wherein the compound is 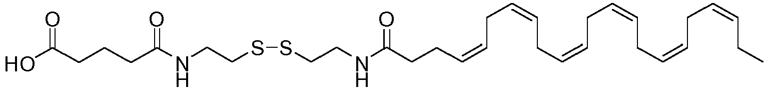 or a pharmaceutically acceptable salt thereof.

5. The compound of claim 1, wherein the compound is 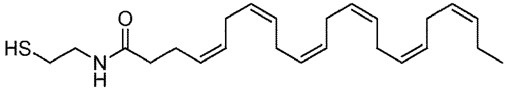 or a pharmaceutically acceptable salt thereof.

6. The compound of claim 1, wherein the compound is 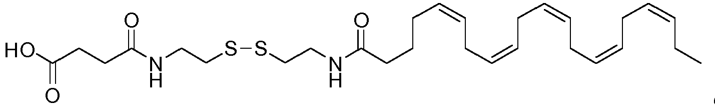 or a pharmaceutically acceptable salt thereof.

7. The compound of claim 1, wherein the compound is 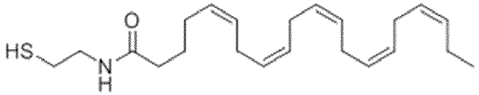 or a pharmaceutically acceptable salt thereof.

8. A pharmaceutical composition comprising a compound of any one of claims 1-7 and a pharmaceutically acceptable excipient.

9. A compound of any one of claims 1-7 or the pharmaceutical composition of claim 8 for use in the treatment of autoimmune disease, inflammatory respiratory disease, or neurodegenerative disease, preferably wherein the autoimmune disease is selected from cystic fibrosis, rheumatoid arthritis, psoriasis, systemic lupus erythematosus, and inflammatory bowel disease, or wherein the inflammatory respiratory disease is selected from asthma, adult respiratory distress syndrome, chronic obstructive airway disease, COPD and cystic fibrosis, or wherein the neurodegenerative disease is selected from multiple sclerosis, Parkinson's disease, Alzheimer's disease, Huntington's disease, amyotrophic lateral sclerosis (ALS) and muscular dystrophy.

## Patentansprüche

1. Eine Verbindung mit der Formel I: 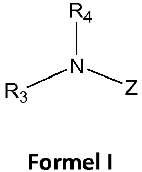 oder ein pharmazeutisch annehmbares Salz, Enantiomer oder Stereoisomer davon; wobei 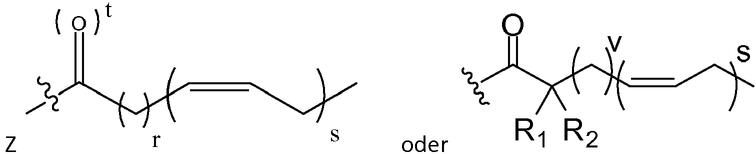 ist;
jedes r unabhängig 2, 3 oder 7 ist;
jedes s unabhängig 3, 5 oder 6 ist;
jedes t unabhängig 0 oder 1 ist;
jedes v unabhängig 1, 2 oder 6 ist:
R₁ und R₂ unabhängig -H, -D, -C₁-C₄ Alkyl, -Halogen, -OH, -C(O)C₁-C₄ Alkyl, , O-Aryl, O-Benzyl, -OC(O)C₁-C₄ Alkyl, -C₂-C₃ Alken, -C₂-C₃ Alkin, -NH₂, -NH(C₁-C₃ alkyl), -N(C₁-C₃ Alkyl)₂, -NH(C(O)C₁-C₃ Alkyl), -N(C(O)C₁-C₃ Alkyl)₂, -SH, -S(C₁-C₃ Alkyl), -S(O)C₁-C₃ Alkyl, oder -S(O)₂C₁-C₃ Alkyl ist;
R₃ H ist
und R₄ ausgewählt ist aus der Gruppe bestehend aus 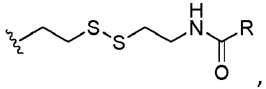 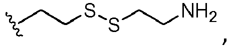 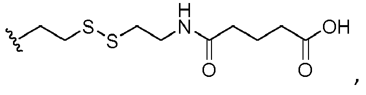 und 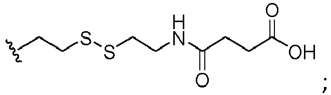 wobei
R ein unverzweigtes oder verzweigtes unsubstituiertes -C₁-C₆ Alkyl ist; oder
eine Verbindung ist ausgewählt aus 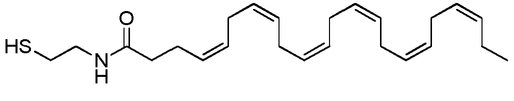 und 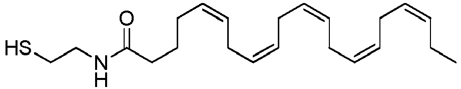 oder ein pharmazeutisch annehmbares Salz davon.

2. Die Verbindung nach Anspruch 1, wobei die Verbindung 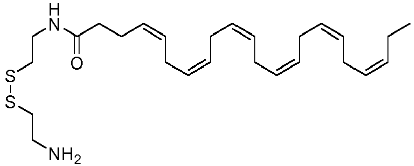 ist oder ein pharmazeutisch annehmbares Salz davon.

3. Die Verbindung nach Anspruch 1, wobei die Verbindung 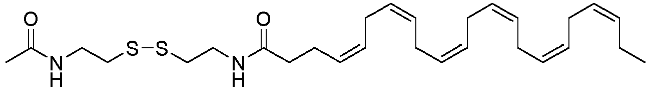 ist oder ein pharmazeutisch annehmbares Salz davon.

4. Die Verbindung nach Anspruch 1, wobei die Verbindung 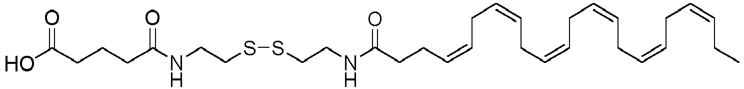 ist oder ein pharmazeutisch annehmbares Salz davon.

5. Die Verbindung nach Anspruch 1, wobei die Verbindung 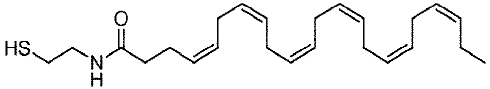 ist oder ein pharmazeutisch annehmbares Salz davon.

6. Die Verbindung nach Anspruch 1, wobei die Verbindung 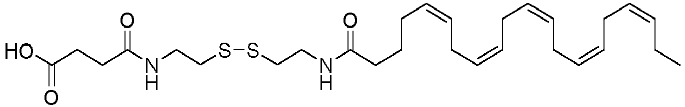 ist oder ein pharmazeutisch annehmbares Salz davon.

7. Die Verbindung nach Anspruch 1, wobei die Verbindung 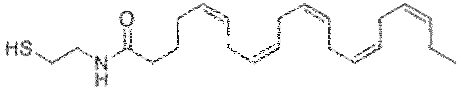 ist oder ein pharmazeutisch annehmbares Salz davon.

8. Eine pharmazeutische Zusammensetzung, umfassend eine Verbindung nach einem der Ansprüche 1-7 und einen pharmazeutisch annehmbaren Trägerstoff.

9. Eine Verbindung nach einem der Ansprüche 1-7 oder die pharmazeutische Zusammensetzung nach Anspruch 8 zur Verwendung bei der Behandlung einer Autoimmunerkrankung, einer entzündlichen Atemwegserkrankung oder einer neurodegenerativen Erkrankung, bevorzugt wobei die Autoimmunerkrankung ausgewählt ist aus Cystischer Fibrose, Rheumatoider Arthritis, Psoriasis, Systemischer Lupus Erythematodes und chronisch entzündlicher Darmerkrankung, oder wobei die entzündliche Atemwegserkrankung ausgewählt ist aus Asthma, Adultem Atemnotsyndrom, Chronisch obstruktive Lungenerkrankung, COPD und Cystischer Fibrose, oder wobei die neurodegenerative Erkrankung ausgewählt ist aus Multipler Sklerose, Parkinson, Alzheimer, Chorea Huntington, Amyotrophe Lateralsklerose (ALS) und Muskuläre Dystrophie.

## Revendications

1. Composé de formule I : 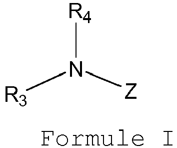 ou un sel, un énantiomère ou un stéréoisomère pharmaceutiquement acceptables de celui-ci ;
dans lequel
Z est 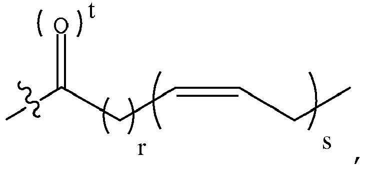 ou 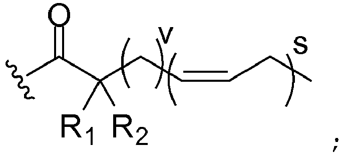
chaque r est indépendamment 2, 3, ou 7 ;
chaque s est indépendamment 3, 5, ou 6 ;
chaque t est indépendamment 0 ou 1 ;
chaque v est indépendamment 1, 2, ou 6 ;
R₁ et R₂ sont indépendamment -H, -D, -alkyle en C₁-C₄, -halogène, -OH, -C(O) (alkyle en C₁-C₄) , -O-aryle, -O-benzyle, -OC(O) (alkyle en C₁-C₄), -alcène en C₂-C₃, -alcyne en C₂-C₃, -NH₂, -NH (alkyle en C₁-C₃), -N (alkyle en C₁-C₃)₂, -NH(C(O) (alkyle en C₁-C₃)), -N(C(O) (alkyle en C₁-C₃))₂, -SH, -S (alkyle en C₁-C₃), -S(O) (alkyle en C₁-C₃), ou -S(O)₂ (alkyle en C₁-C₃) ;
R₃ est H
et R₄ est sélectionné dans le groupe constitué par 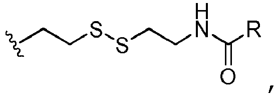 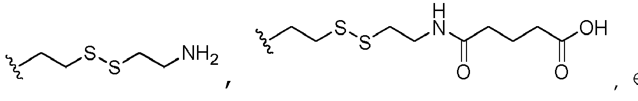 et 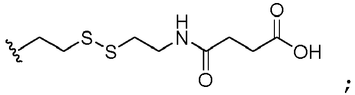 dans lequel
R est un groupe -alkyle en C₁-C₆ non substitué linéaire ou ramifié ; ou
un composé sélectionné parmi 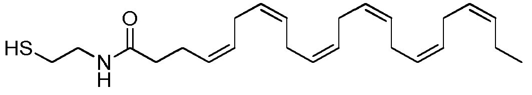 et 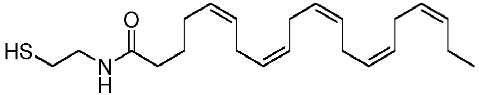 ou un sel pharmaceutiquement acceptable de celui-ci.

2. Composé selon la revendication 1, dans lequel le composé est 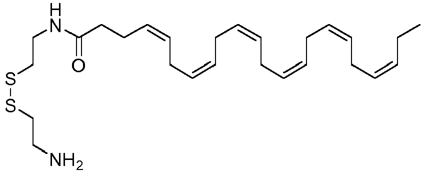 ou un sel pharmaceutiquement acceptable de celui-ci.

3. Composé selon la revendication 1, dans lequel le composé est 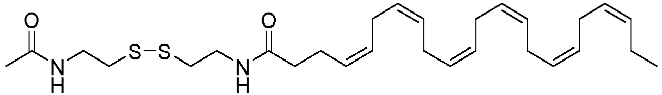 ou un sel pharmaceutiquement acceptable de celui-ci.

4. Composé selon la revendication 1, dans lequel le composé est 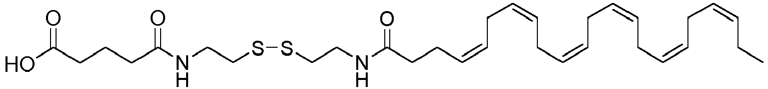 ou un sel pharmaceutiquement acceptable de celui-ci.

5. Composé selon la revendication 1, dans lequel le composé est 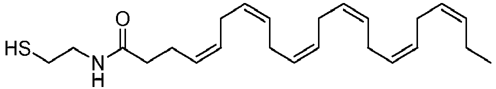 ou un sel pharmaceutiquement acceptable de celui-ci.

6. Composé selon la revendication 1, dans lequel le composé est 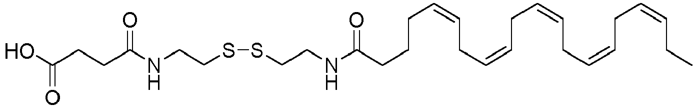 ou un sel pharmaceutiquement acceptable de celui-ci.

7. Composé selon la revendication 1, dans lequel le composé est 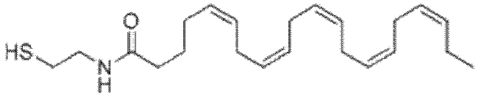 ou un sel pharmaceutiquement acceptable de celui-ci.

8. Composition pharmaceutique comprenant un composé selon l'une quelconque des revendications 1 à 7 et un excipient pharmaceutiquement acceptable.

9. Composé selon l'une quelconque des revendications 1 à 7 ou la composition pharmaceutique selon la revendication 8 pour son utilisation dans le traitement d'une maladie auto-immune, d'une maladie respiratoire inflammatoire, ou d'une maladie neurodégénérative, de préférence dans lequel la maladie auto-immune est sélectionnée parmi la mucoviscidose, la polyarthrite rhumatoïde, le psoriasis, le lupus érythémateux systémique, et une affection abdominale inflammatoire, ou dans lequel la maladie respiratoire inflammatoire est sélectionnée parmi l'asthme, le syndrome de détresse respiratoire de l'adulte, une maladie chronique obstructive des voies respiratoires, la BPCO et la mucoviscidose, ou dans lequel la maladie neurodégénérative est sélectionnée parmi la sclérose en plaques, la maladie de Parkinson, la maladie d'Alzheimer, la chorée d'Huntington, la sclérose latérale amyotrophique (SLA) et la dystrophie musculaire.
